(19)

Europäisches
Patentamt
European
Patent Office
Office européen
des brevets

(11) **EP 4 174 493 A1**

(12) **EUROPEAN PATENT APPLICATION**

(43) Date of publication:
**03.05.2023 Bulletin 2023/18**

(21) Application number: **21205516.4**

(22) Date of filing: **29.10.2021**

(51) International Patent Classification (IPC):
***G01N 33/574*** (2006.01)

(52) Cooperative Patent Classification (CPC):
**G01N 33/57488; G01N 33/57423;**
G01N 2333/70532; G01N 2333/70564;
G01N 2800/52

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Eberhard Karls Universität Tübingen
Medizinische Fakultät
72074 Tübingen (DE)**

(72) Inventors:
• **Zender, Lars
72108 Rottenburg - Wendelsheim (DE)**

• **Hinterleitner, Clemens
72076 Tübingen (DE)**

(74) Representative: **Witte, Weller & Partner
Patentanwälte mbB
Postfach 10 54 62
70047 Stuttgart (DE)**

<u>Remarks:</u>
The complete document including Reference
Table(s) and the Sequence Listing(s) can be
downloaded from the EPO website

(54) **METHOD OF PREDICTING A PATIENT'S BENEFIT FROM THERAPY WITH AN IMMUNE
CHECKPOINT INHIBITOR**

(57)    The present invention relates to a method for predicting a patient's benefit from therapy with an immune checkpoint inhibitor, a method for predicting a cancer patient's probability of survival, and a method for determining in a sample the value of an expression level of a platelet surface protein.

**EP 4 174 493 A1**

**Description**

The present invention relates to a method of predicting a patient's benefit from therapy with an immune checkpoint inhibitor, a method of predicting a cancer patient's probability of survival, and a method for determining in a sample the value of an expression level of a platelet surface protein.

FIELD OF THE INVENTION

The present invention relates to the field of molecular biology and molecular medicine, more particular to the field of molecular diagnostics and prognostics.

BACKGROUND OF THE INVENTION

Immune checkpoint receptors like CTLA4 and PD-1 are crucial for preventing excessive immune responses and autoimmunity. Seminal discoveries made by Allison and Honjo provided preclinical proof of concept data that blockage of CTLA4 and PD-1 signaling unleashes marked anti-tumor immune responses. Clinical evaluation revealed remarkable therapeutic potential of immune checkpoint inhibition in human cancer patients and for the first time allowed for long term survival of patients with advanced metastasized solid tumors. Besides melanoma patients, especially patients suffering from non-small cell lung cancer (NSCLC) benefit from treatment with antibodies inhibiting the PD-1 and CTLA4 immune checkpoints. Nevertheless, simple and robust biomarkers to predict therapy responses towards immune checkpoint inhibitor (ICI) are still missing.

With 1.8 million deaths per year, lung cancer represents one of the most frequent and lethal cancers worldwide. In the US 254,170 new lung cancer cases are expected to be diagnosed in 2021. Given the high frequency of lung cancer and the cost of checkpoint inhibitory therapies, the lack of robust biomarkers to select patients who best possibly benefit from ICI represents a major burden for the health systems.

Histological quantification of intratumoral PD-L1 expression is routinely performed in an attempt to predict therapy responses towards ICI, however, only an insufficient correlation between detection of PD-L1 expression in tumor biopsies and the overall response rate (ORR) was found (see Teng F., Meng X., Kong L., Yu J. Progress and challenges of predictive biomarkers of anti PD-1/PD-L1 immunotherapy: A systematic review. Cancer letters. 2018;414:166-173).

In lung cancer, evaluation of smoking history, tumor mutational burden (TMB), microsatellite instability (MSI), high expression of CTLA4, low expression of CX3CL1 and infiltration of CD8$^+$ T cells within the tumor microenvironment (TME) seems to be superior in predicting therapy responses towards anti-PD-1/PD-L1 directed ICI when compared to histopathological PD-L1 quantification, however these markers so far could not be translated into a robust and clinically easy to use biomarker signature.

Interestingly, recent research results have shown that tumor-specific protein markers are not only present freely in the blood, but can also be taken up by platelets, so-called thrombocytes, which classically mediate hemostasis. Platelets express these protein markers, so that after their isolation an assessment on the course of tumor diseases can be made.

However, a distinctive feature of platelet surface protein expression is that a large number of them are regulated depending on the activation state of the platelet. For example, some proteins are stored preformed in intracellular vesicles and are transported to the cell surface upon platelet activation. Accordingly, the amount of protein on the surface increases in the activated state. In contrast, so-called matrix metalloproteases (MMPs) in activated platelets can cut proteins from the surface (so-called shedding), which reduces the total amount of protein on the platelet surface after activation.

However, since platelets exhibit different activation levels both during sample collection and in the organism due to various stimuli (negatively charged surfaces in the sample vessel, shear forces during blood collection, inflammatory mediators, etc.) and protein expression depends on the activation state, an exact determination of total protein expression on the platelet surface is difficult. Thus, platelet activation status acts as a confounding factor that reduces the sensitivity and specificity of platelet surface protein expression as a biomarker.

Against this background it is an object underlying the invention to provide a method for predicting a patient's benefit from therapy with an ICI, which involves the use of an appropriate biomarker. It is also an object underlying the invention to provide a method for predicting a cancer patient's probability of survival, where the prediction is based on an appropriate biomarker. By means of said methods the disadvantages of the state of the art are avoided or at least significantly reduced.

The present invention satisfies these and other needs.

SUMMARY OF THE INVENTION

The present invention provides a method of predicting a patient's benefit from therapy with an immune checkpoint inhibitor (ICI), comprising the steps of:

1) Providing a platelets-containing sample from the patient;

2) Determining the expression levels on the platelets' surface of:

- platelet programmed cell death 1 ligand 1 (pPD-L1) to obtain pPD-L1$^{expr.}$, and

- a platelet activation marker (A) to obtain A$^{expr.}$;

3) Determining a correction value pPD-L1$^{corr.}$ as follows:

- Providing a matrix with $m$ rows and $n$ columns, the elements $a_{ij}$ for the $i$-th row and the $j$-th column, where each row $i$ is assigned to an expression level of A and each column j is assigned to an expression level of pPD-L1,

- selecting the element $a_{ij}$ from the matrix based on the expression levels determined in step 2 to obtain pPD-L1$^{corr.}$;

4) Determining an adjusted expression level on the platelet surface of pPD-L1 to obtain pPD-L1$^{adj.}$ as follows:

$$pPD\text{-}L1^{expr.} + pPD\text{-}L1^{corr.} = pPD\text{-}L1^{adj.};$$

5) Predicting

- a therapeutic benefit if pPD-L1$^{adj.} \geq$ reference value x, or

- no therapeutic benefit if pPD-L1$^{adj.} <$ reference value x.

According to the invention, an "immune checkpoint inhibitor" (ICI) refers to a molecule that inhibits an immune checkpoint, i.e. key regulators of the immune system that when stimulated can dampen the immune response to an immunologic stimulus. ICIs can block inhibitory checkpoints, restoring immune system function. Currently approved checkpoint inhibitors target the molecules CTLA4, PD-1, and PD-L1. Typical immune checkpoint inhibitors are e.g. antibodies against CTLA-4 (e.g. ipilimumab), PD-1 (e.g. nivolumab) and PD-L1 (e.g. atezolizumab, durvalumab and avelumab). After the ICI has been infused, the ICI binds to these proteins, which act as immune checkpoints. As a result, the cells that carry one of these proteins on the cell surface and bind the ICI are temporarily (or as long as the therapeutic ICI is circulating in the body) attacked by immune cells and removed from the body by macrophages (temporary cell depletion). These processes lead to an intensification of the immune response against the tumor, so that its strategy of immune evasion is counteracted.

According to the invention a "platelets-containing sample" refers to any solution, liquid or semi-liquid sample, which contain thrombocytes or blood platelets, respectively, such as a blood sample, a buffered platelets solution, a solution of biological cells etc. The term platelet and thrombocyte can be used interchangeably.

According to the invention the expression level is a measure of the strength of the presence of the analyzed protein molecule in the platelets, i.e. the occurrence of the amount of protein exposed on the platelets' surface. In an embodiment of the invention the level of expression is indicated as the percentage of platelets which carry the analyzed protein molecule on their surfaces in proportion to all analyzed platelets. In a preferred embodiment of the invention the expression level of the protein molecule is determined via flow cytometry or fluorescence-activated cell sorting (FACS), respectively.

According to the invention "pPD-L1$^{expr.}$" refers to the expression value or level of pPD-L1 as determined in step 2) of the method according to the invention.

According to the invention, "platelet programmed cell death 1 ligand 1" (pPD-L1), also known as cluster of differentiation 274 (CD274) or B7 homolog 1 (B7-H1), is a protein that in humans is encoded by the CD274 gene (Human = Entrez 29126; Ensembl: ENSG00000120217; UniProt: Q9NZQ7). Programmed death-ligand 1 (PD-L1) is a 40kDa type 1 transmembrane protein that has been speculated to play a major role in suppressing the adaptive arm of immune systems during particular events such as pregnancy, tissue allografts, autoimmune disease and other disease states such as hepatitis. The binding of PD-L1 to the inhibitory checkpoint molecule PD-1 transmits an inhibitory signal based on interaction with phosphatases (SHP-1 or SHP-2) via immunore-ceptor tyrosine-based switch motif (ITSM). This reduces the proliferation of antigen-specific T-cells in lymph nodes, while simultaneously reducing apoptosis in regulatory T cells (anti-inflammatory, suppressive T cells) - further mediated by a lower regulation of the gene Bcl-2.

According to the invention, a "platelet activation marker" or "A" refers to an indicator molecule which signals the activated status of the platelets. Platelet or thrombocyte activation is a complex morphological and biochemical change of platelets at the site of a vascular lesion, that initiates hemostasis. It is triggered primarily by the contact of platelet glycoproteins with subendothelial collagen fibers and von Willebrand factor. Examples of A include CD62P (P-selectin) and PAC-1 (first procaspase activating compound).

In the first sub-step of step 3) of the method according to the invention a matrix is provided having the following format:

$$
\begin{array}{c}
\textbf{matrix} \quad \textsf{m} \times \textsf{n} \\
a_{ij} \quad \text{n columns} \quad \text{j runs} \\
\text{m rows} \quad i \; runs
\end{array}
\qquad
\begin{pmatrix}
a_{11} & a_{12} & a_{13} & \cdots \\
a_{21} & a_{22} & a_{23} & \cdots \\
a_{31} & a_{32} & a_{33} & \cdots \\
\vdots & \vdots & \vdots & \ddots \\
 & & & a_{mn}
\end{pmatrix}
$$

Each row $i$ is assigned to an expression level or value of A, e.g. the most upper row represents a high expression level of A, and, e.g., the most lower row represents a low expression level or value of A. Each column $j$ is assigned to an expression level of pPD-L1, e.g. the most left column represents a low expression level or value of pPD-L1, and, e.g., the most right column represents a high expression level or value of pPD-L1.

In the second sub-step of step 3) of the method according to the invention the element $a_{ij}$ is selected from the matrix based on the expression levels determined in step 2), i.e. $A^{expr.}$ and pPD-L1$^{expr.}$. To do this, select row $i$, which indicates the value or range of values of A corresponding to $A^{expr.}$, and select column $j$, which indicates the value or range of values of pPD-L1 corresponding to pPD-L1$^{expr.}$. Then determine from the pair of values the corresponding pPD-L1$^{corr.}$ from the matrix.

According to the invention, in step 4) the values of pPD-L1$^{expr.}$ and pPD-L1$^{corr.}$ are added to result in pPD-L1$^{adj.}$.

In step 5) of the method according to the invention the prediction of the responsiveness of the patient to the ICI therapy is made. The inventors have found out that there will be a therapeutic benefit if pPD-L1$^{adj.}$ is equal or superior to a reference value x, and there will be less or even no therapeutic benefit if pPD-L1$^{adj.}$ is lower than a reference value x. The reference value x can be empirically determined by the skilled person by taking the median of pPD-L1$^{adj.}$ in the entire reference cohort.

The object underlying the invention is herewith fully achieved.

The inventors have realized that by means of the provided method an exact determination of a platelet expressed biomarker, pPD-L1, can be realized, independent of the activation state of the platelets. This is made via determining a corrected expression level pPD-L1$^{corr.}$ of pPD-L1. By adding pPD-L1$^{corr.}$ to the actual measured value of pPD1 the adjusted expression level pPD-L1$^{adj.}$ is obtained. Once this latter value is equal to or exceeds a threshold or reference value x, the patient has a positive prognosis for a ICI therapy.

As a consequence, the method according to the invention helps to identify the kinds of patients which are very likely to have a good responsiveness to an ICI therapy and, therefore, those patients which benefit best from such a therapy. On the other hand, the method allows excluding those patients from an ICI therapy which will not have significant benefit from an ICI therapy. It helps to reduce the burden on the healthcare system, as ICI therapy is very expensive. It further prevents patients with a low responsiveness from the side effects which are associated with an ICI therapy. In such a situation the side effects are not in a tolerable relationship with the expected therapy success.

In an embodiment of the method according to the invention, A is CD62P and, consequently, $A^{expr.}$ is CD62P$^{expr.}$.

This measure has the advantage that a particularly suited platelet activation marker is used which finally results in a reliable prognosis of the patient's responsiveness.

CD62, also called P-selectin, granule membrane protein 140 (GMP-140), and platelet activation-dependent granule to external membrane protein (PADGEM), is a type-1 transmembrane protein that in humans is encoded by the SELP gene (Entrez: 6403; Ensembl: ENSG00000174175; UniProt: P16109). CD62P functions as a cell adhesion molecule (CAM) on the surfaces of activated endothelial cells, which line the inner surface of blood vessels, and activated platelets. In inactivated platelets CD62P is stored in $\alpha$-granules.

In another embodiment of the invention in the matrix $n$ is 4 resulting in pPD-L1 quartile groups Q1 (very low), Q2 (low), Q3 (high), and Q4 (very high).

The inventors recognized that subdividing in the matrix the expression levels of pPD-L1 into quartiles is useful and allows a sufficiently accurate prediction of the patient's response rate to ICI therapy.

In yet another embodiment of the method according to the invention in the matrix

- Q1 is assigned to an expression level of pPD-L1 of approx. 0 - 0.3%,

- Q2 is assigned to an expression level of pPD-L1 of approx. 0.4 - 0.9%,

- Q3 is assigned to an expression level of pPD-L1 of approx. 1 - 2%,

- Q4 is assigned to an expression level of pPD-L1 of approx. $\geq$ 2.1%.

According to the inventors' findings, these subdivisions have proven to be expedient and are therefore particularly advantageous.

In another embodiment of the invention in the matrix m is 5 resulting in CD62P expression groups E1, E2, E3, E4, and E5.

It turned out that the subdivisions of the expression levels of CD62P into 5 groups is adequate and results in a proper process of determining of pPD-L1$^{corr.}$.

In still another embodiment of the method according to the invention in the matrix

- E1 is assigned to an expression level of CD62P of approx. >80 - 100%,

- E2 is assigned to an expression level of CD62P of approx. >60 - 80%,

- E3 is assigned to an expression level of CD62P of approx. >40 - 60%,

- E4 is assigned to an expression level of CD62P of approx. >20 - 40%,

- E5 is assigned to an expression level of CD62P of approx. 0 - 20%.

The inventors realized that the allocation of the indicated expression ranges is reasonable for an accurate determination of pPD-L1$^{corr.}$.

In another embodiment of the invention the elements $a_{ij}$ of the matrix are as follows:

$a_{11} = 0$; $a_{12} = 0.10$; $a_{13} = 1.10$; $a_{14} = 2.14$;

$a_{21} = 0.01$; $a_{22} = 0.12$; $a_{23} = 1.57$; $a_{24} = 3.50$;

$a_{31} = 0.20$; $a_{32} = 0.81$; $a_{33} = 2.53$; $a_{34} = 4.70$;

$a_{41} = 0.37$; $a_{42} = 1.40$; $a_{43} = 5.43$; $a_{44} = 4.71$;

$a_{51} = 0.60$; $a_{52} = 1.12$; $a_{53} = 2.34$; $a_{54} = 3.14$.

As a result, the matrix according to the invention looks as follows:

The values of the respective $a_{ij}$ were empirically determined by the inventors and turned out to allow an accurate determination of the correction value pPD-L1$^{corr.}$.

In a preferred embodiment of the invention the reference value x is 2.1%.

The reference value of x = 2.1% has been identified by the inventors as being of particular preference because it allows a reliable determination of the responsiveness of the patients to ICI therapy.

In another embodiment of the invention, the expression level of pPD-L1$^{expr.}$ and/or A$^{expr.}$ is determined via flow cytometry, preferably via fluorescence-activated cell sorting (FACS).

The use of flow cytometry or FACS allows an accurate and a time saving determination of the expression levels of pPD-L1$^{expr.}$ and A$^{expr.}$. These techniques enable an implementation of the method according to the invention in the everyday routine of clinical laboratories.

In another embodiment of the invention said platelets-containing sample is a blood sample.

This measure has the advantage that the optimum sample of platelets is used, which routinely occurs anyway with the patients being treated. Therefore, performing the method according to the invention does not impose any additional burden on the patient.

In yet another embodiment of the invention said immune ICI is selected from the group consisting of: pembrolizumab, nivolumab, ipilimumab, tremelimumab, cemiplimab, spartalizumab, atezolizumab, durvalumab, and avelumab.

By this measure the measure according to the invention is adapted to the ICIs which are currently available. This embodiment, therefore, implements the method into the common ICI therapies.

In a still further embodiment of the invention said patient is suffering from non-small cell lung cancer (NSCLC).

This measure has the advantage that the method according to the invention is applied to a cancer disease which is currently treated with ICIs, and in which the method according to the invention has been shown to allow a particularly reliable prediction of therapeutic success.

Another subject-matter of the invention relates to a method for predicting a cancer patient's probability of survival, comprising the steps of:

1) Providing a platelets-containing sample from the patient;

2) Determining the expression levels on the thrombocytes' surface of:

- platelet programmed cell death 1 ligand 1 (pPD-L1) to obtain pPD-L1$^{expr.}$, and

- a platelet activation marker (A) to obtain A$^{expr.}$;

3) Determining a correction value pPD-L1$^{corr.}$ as follows:

- Providing a matrix with $m$ rows and $n$ columns, the elements $a_{ij}$ for the $i$-th row and the $j$-th column, where each row $i$ is assigned to an expression level of A and each column $j$ is assigned to an expression level of pPD-L1,

- selecting the element $a_{ij}$ from the matrix based on the expression levels determined in step 2 to obtain pPD-L1$^{corr.}$;

4) Determining an adjusted expression level on the platelet surface of pPD-L1 to obtain pPD-L1$^{adj.}$ as follows:

$$\text{pPD-L1}^{expr.} + \text{pPD-L1}^{corr.} = \text{pPD-L1}^{adj.};$$

5) Predicting

- a low probability of survival if pPD-L1$^{adj.} \geq$ reference value x, or

- a high probability of survival if pPD-L1$^{adj.} <$ reference value x.

The features, characteristics, advantages and embodiments of the therapy-responsiveness prediction method according to the invention mentioned at the outset apply - *mutatis mutandis* - also to the present cancer-progression prediction method. In contrast to the therapy prediction method in step 5) a poor survival prognosis is diagnosed if pPD-L1$^{adj.}$ is equal or superior to the reference value x, and a good survival prognosis is diagnosed if pPD-L1$^{adj.}$ is lower than the reference value x.

Still another subject-matter of the present invention relates to a method for determining in a sample the value of an expression level of a platelet surface protein, said expression level being independent of the activation state of the platelet ($p^{ind.}$), said method comprising the following steps:

1. Providing a platelets-containing sample,

2. Determining on said platelets the expression level of:

- a platelet surface protein p to obtain $p^{expr.}$, and

- a platelet activation marker A to obtain $A^{expr.}$;

3. Determining a correction value $p^{corr.}$ as follows:

- Providing a matrix with $m$ rows and $n$ columns, the the elements $a_{ij}$ for the $i$-th row and the $j$-th column, where each row $i$ is assigned to an expression level of said platelet activation marker and each column $j$ is assigned to an expression level of said platelet surface protein,

- selecting the element $a_{ij}$ from the matrix based on $p^{expr.}$ and $A^{expr.}$ determined in step 2 to obtain $p^{corr.}$;

4. Determining $p^{ind.}$ as follows:

$$p^{expr.} + p^{corr.} = p^{ind.}$$

The features, characteristics, advantages and embodiments of the therapy-responsiveness prediction method according to the invention mentioned at the outset apply - *mutatis mutandis* - also to the present expression determining method.

The inventors have realized that the principles embodied in the therapy-responsiveness prediction method with respect to pPD-L1 can be likewise applied to other platelet surface proteins to determine the "real" expression level of said platelet surface proteins.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

BRIEF DESCRIPTION OF THE FIGURES

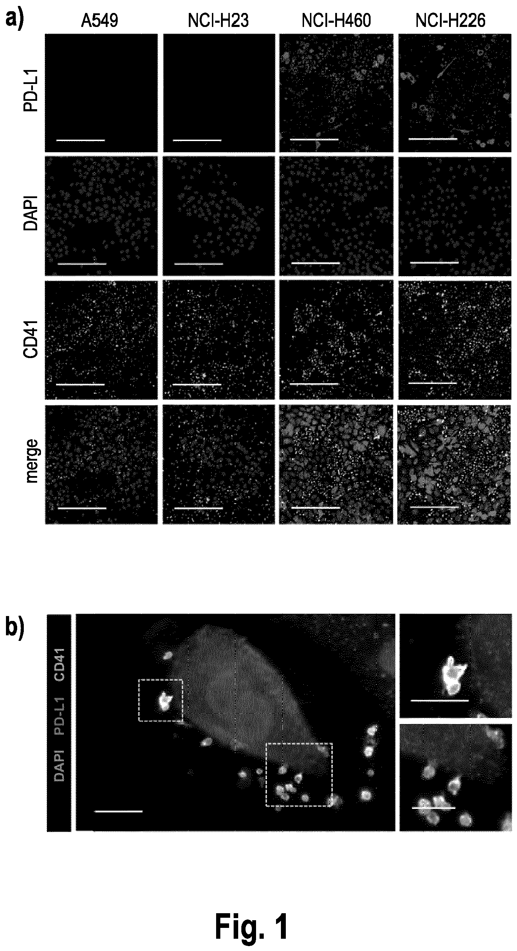

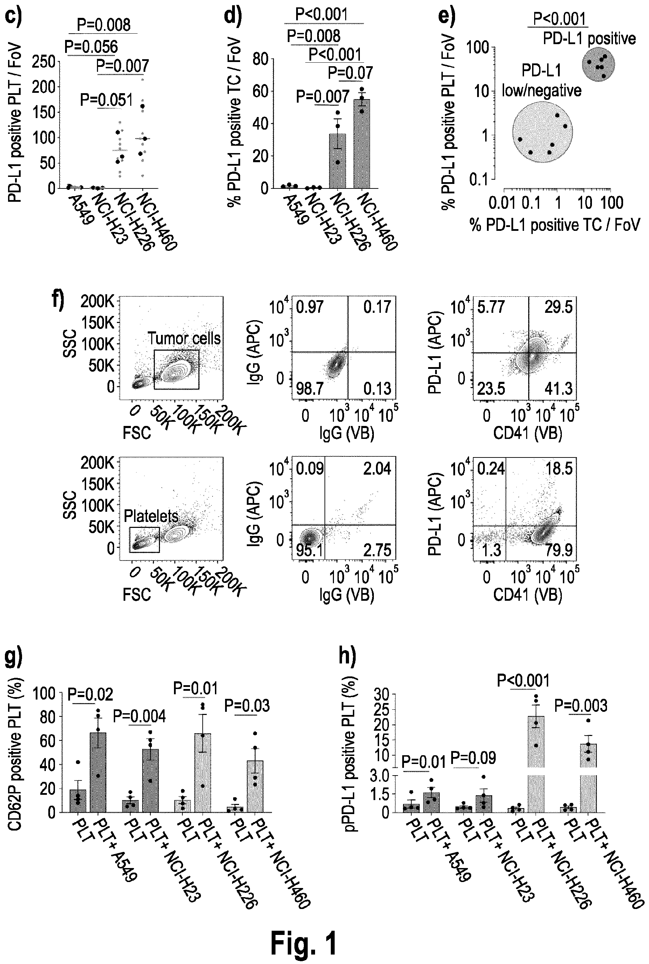

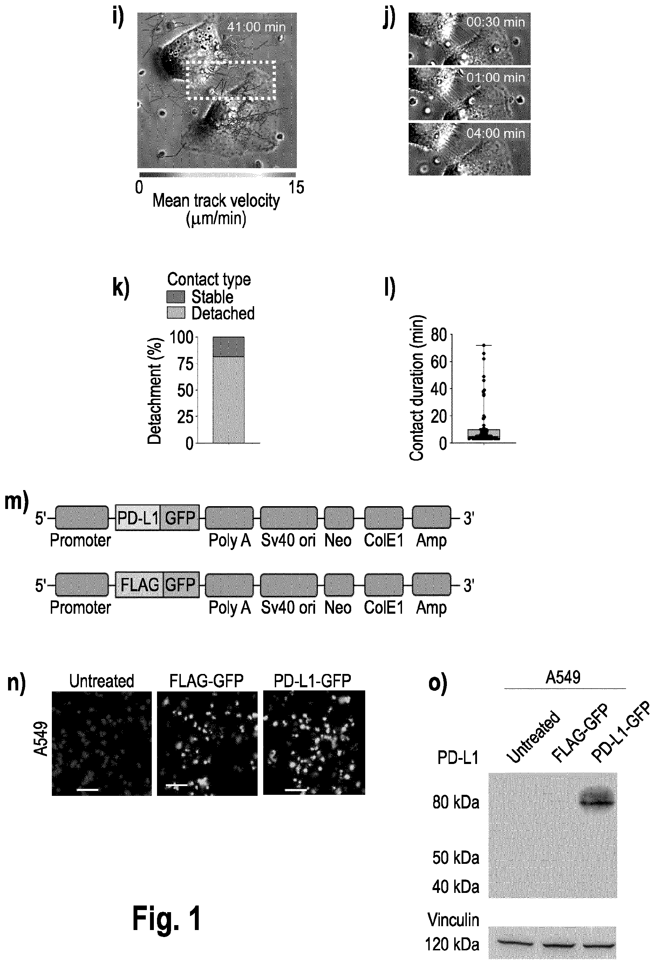

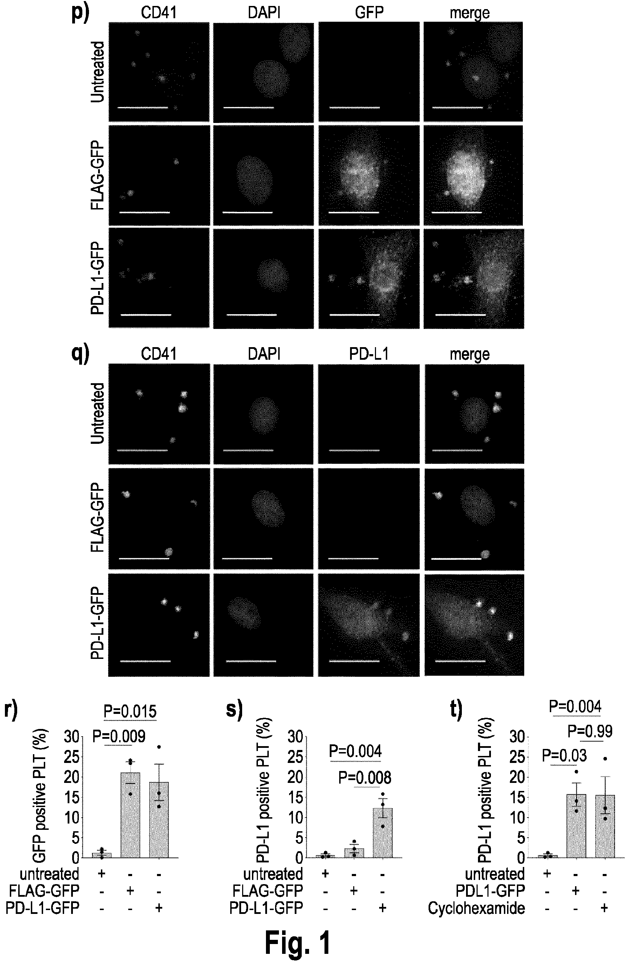

Fig. 1: Direct platelet-tumor cell interactions with tumor cells increase pPD-L1 protein levels on the platelet surface. a Representative immunofluorescence staining of PD-L1 (red), DAPI (blue) and the platelet marker CD41 (green) in four different NSCLC tumor cell lines (A549, NCI-H23, NCI-H226, NCI-H460) co-incubated with human platelets (n = 3 biological replicates). Scale bars, 200 $\mu$m b Immunofluorescence microscopy of NCI-H460 cells interacting with human platelets derived from a healthy donor (PD-L1 in red, CD41 in green) (n = 3). Right scale bar, 20 $\mu$m, left scale bars, 10 $\mu$m. c Quantitative analysis of the PD-L1 positive platelets per field of view (FoV), analysed by immunofluorescence microscopy (n = 9 FoV (small symbols) were analysed out of a total of n=3 independent experiments (large symbols)). Horizontal lines represent means. Statistical significance was calculated by ANOVA and Tukey's multiple comparisons test. d Percentage of PD-L1 positive tumor cells per FoV (n = 3). Data are mean $\pm$ SEM. Statistical significance was calculated by ANOVA and Tukey's multiple comparisons test. e Correlation of % PD-L1 positive platelets / FoV vs. % PD-L1 tumor cells / FoV (n = 3). Correlation was determined by simple linear regression analysis. f Flow cytometry gating strategy for the quantification of PD-L1 positive tumor cells and platelets after co-incubation. g-h Surface expression of PD-L1 and CD62P on control platelets (PLT) and platelets after co-incubation with A549, NCI-H23, NCI-H226, NCI-H460 cells (n = 4). Data are mean $\pm$ SEM. Statistical significance was calculated by Student's t-test. i Phase-contrast image of A549 tumor cells after 41 min coculture with platelets (ratio 1:1000). Overlaid migration tracks were color-coded based on their mean velocity. j Image sequence depicting single platelet interaction with tumor cell and tumor cell protrusion followed by detachment derived from zoom-in area indicated in a. k Percentage of stable platelet-tumor cell contacts lasting from contact initiation until the end of the observation period (total observation time: 41 min). l Contact duration of platelet - tumor cell interactions. Data derived from the analysis of 75 platelets of N = 1 experiments. m Scheme of vectors expressing PD-L1-GFP and FLAG-GFP used for transfection. n Immunofluorescence images of A549 cells transfected with FLAG-GFP or PD-L1-GFP (n = 3). Scale bar 50 $\mu$m. o Western blot analysis for PD-L1 in untreated and transfected A549 cells (n = 2). Vinculin was used as loading control. p-q Representative immunofluorescence microscopy of untreated, FLAG-GFP and PD-L1-GFP transfected A549 cells interacting with platelets (n = 3). Tumor cells and platelets were stained with regard to GFP (upper) and PD-L1 (lower). Scale bar 50 $\mu$m. r-s, Flow-cytometry based quantification of GFP (l) and PD-L1 (m) on platelet surfaces after co-incubation with untreated and transfected A549 cells (n = 3). t Expression of PD-L1 on platelets pre-treated with 100 $\mu$M cycloheximide after co-incubation with PD-L1-GFP transfected A549 cells (n = 3). r-t, Data are mean $\pm$ SEM. Statistical significance was calculated by ANOVA and Tukey's multiple comparisons test.

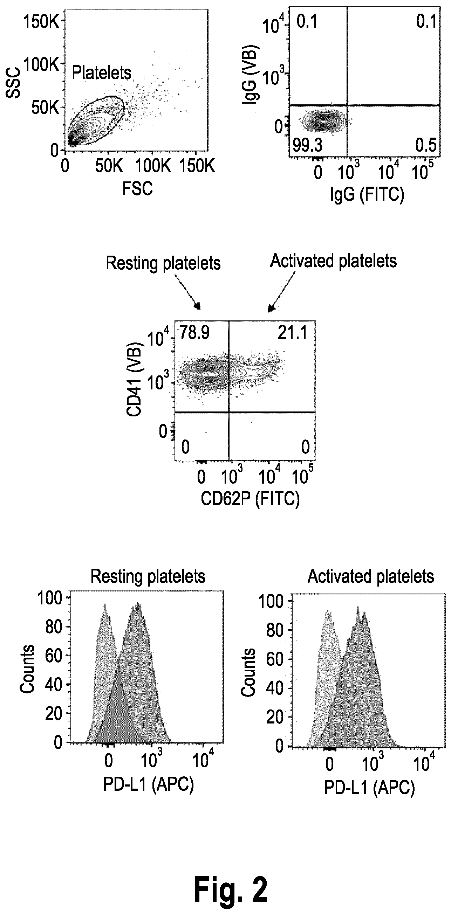

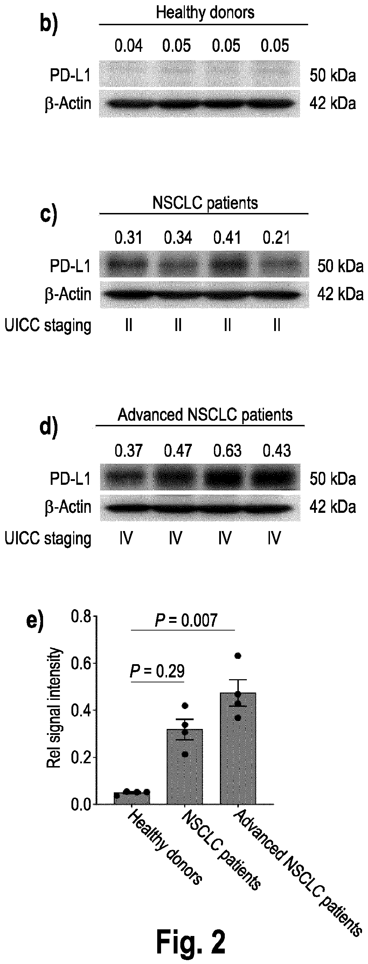

Fig. 2: PD-L1 expression on platelets. a Flow cytometry gating strategy showing the PD-L1 expression on resting and activated platelets of a NSCLC patient (n=3). b Western blots analysis of platelet whole cell lysates showing PD-L1 in healthy donors. $\beta$-actin was used as loading control (n = 4). c-d Western blots analysis showing PD-L1 in NSCLC patients with intermediate (c) and high tumor stages (d) (n = 8). e Quantification of PD-L1 in platelet whole cell lysates of heathy donors and NSCLC patients (n = 12). Data are mean $\pm$ SEM. Statistical significance was calculated by Kurskal-Wallis test and Dunn's multiple comparisons test.

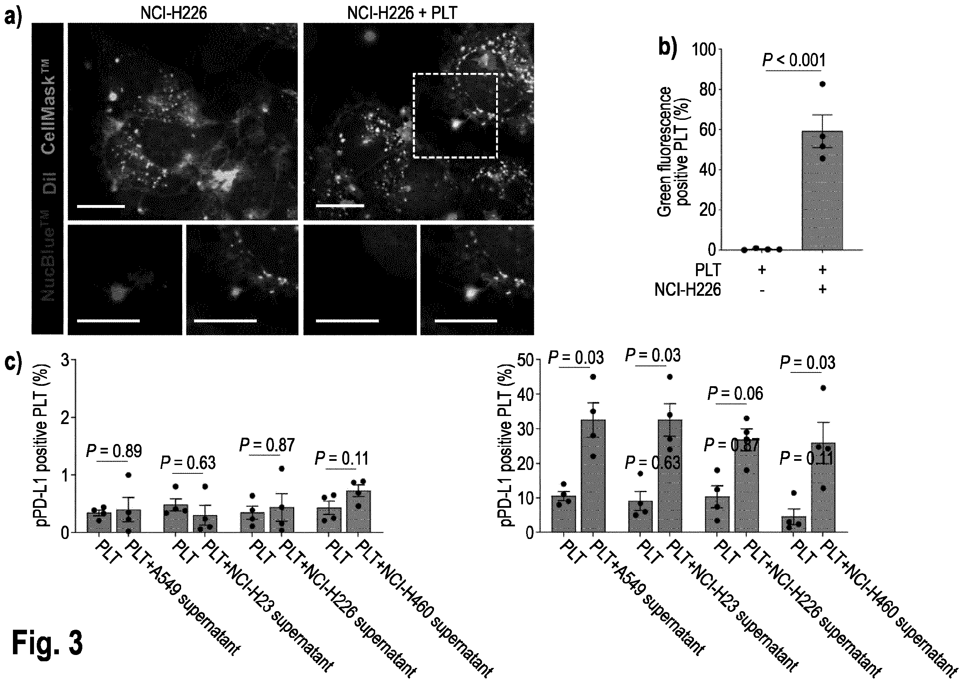

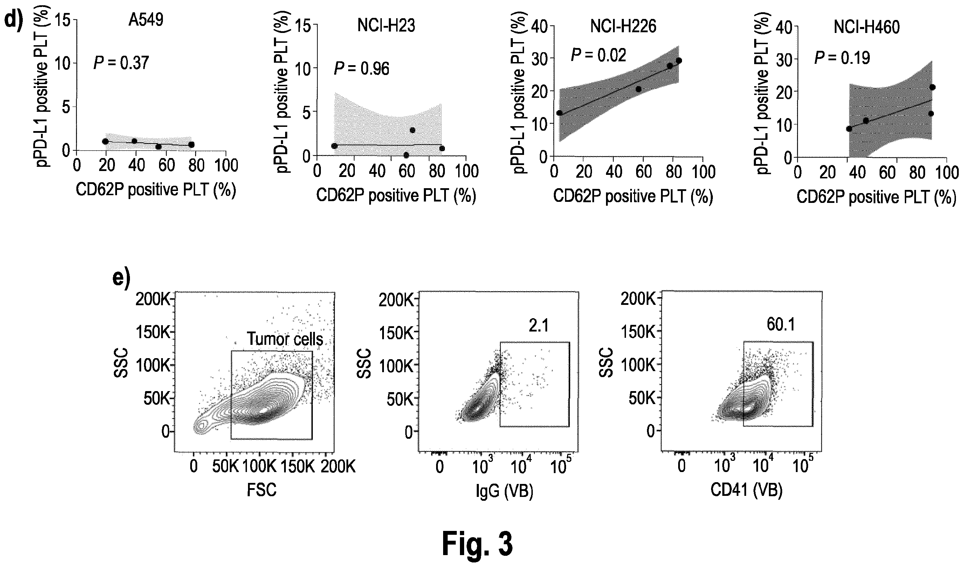

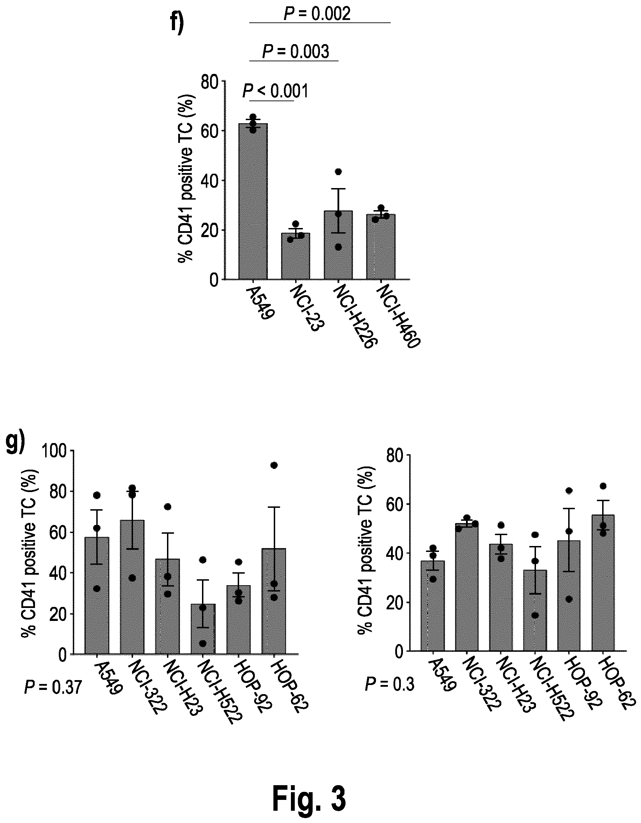

Fig. 3: Direct platelet-tumor cell interactions but not supernatant enables protein exchange. a Membrane transfer of NCI-H226 cells with platelets. Immunofluorescence of lipid membranes (CellMaskTM) in NCI-H226 cells in green. Platelets were counter-stained using DiI (red). Nuclei were stained using NucBlueTM (n = 4). Scale bars, 20 $\mu$m. b Percentage of green fluorescence positive platelets after co-incubation with green labelled NCI-H226 cells quantified via flow cytometry (n = 4). Data are mean $\pm$ SEM. Statistical significance was calculated by Student's t-test. c Surface expression of PD-L1 and CD62P on control platelets (PLT) and platelets after co-incubation with supernatant derived from A549, NCI-H23, NCI-H226, NCI-H460 cells (n = 4). Data are mean $\pm$ SEM. Statistical significance was calculated by Student's t-test. d Correlation of pPD-L1 expression and platelet activation (CD62P expression) on platelets after co-incubation with A549, NCI-H23, NCI-H226, NCI-H460 cells (n = 4). Correlation was determined by simple linear regression analysis. e Flow cytometry gating strategy for the quantification of platelets bound to A549 tumor cells after co-incubation.

f Quantification of platelets bound to tumor cells (n = 3). Data are mean ± SEM. Statistical significance was calculated by Student's t-test. g Quantification of the transfection efficacy of FLAG-GFP and PD-L1-GFP transfected tumor cells (n = 3). Data are mean ± SEM. Statistical significance was calculated by ANOVA and Tukey's multiple comparisons test.

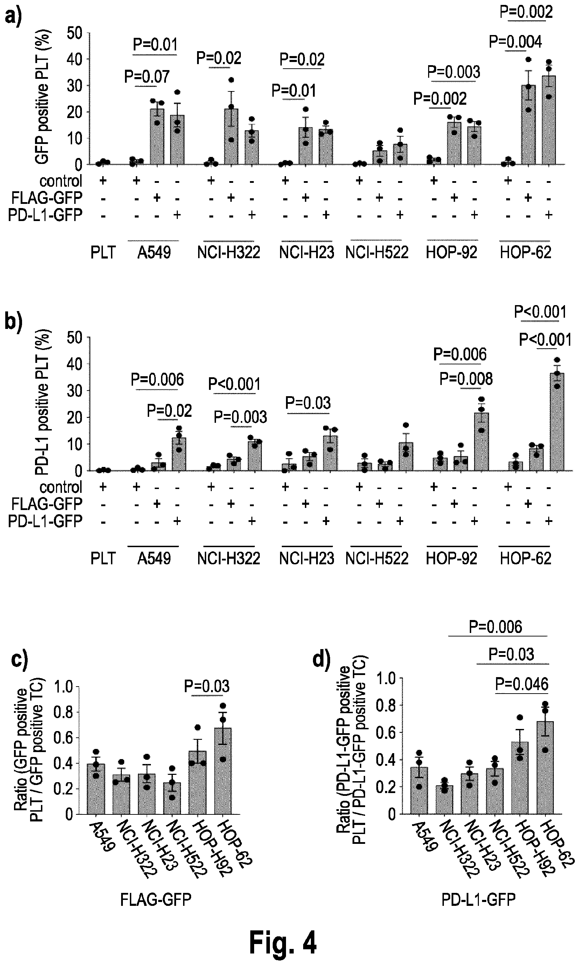

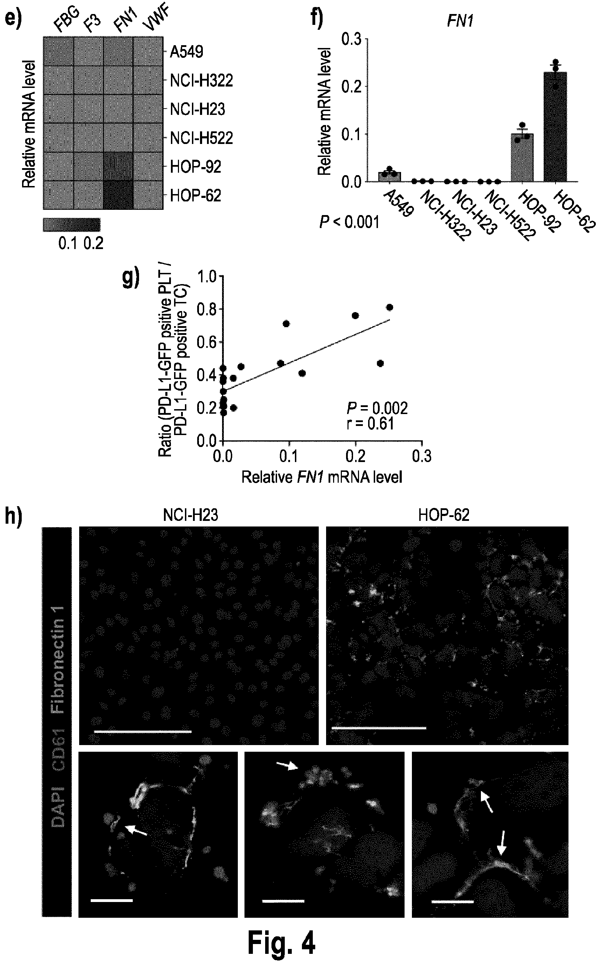

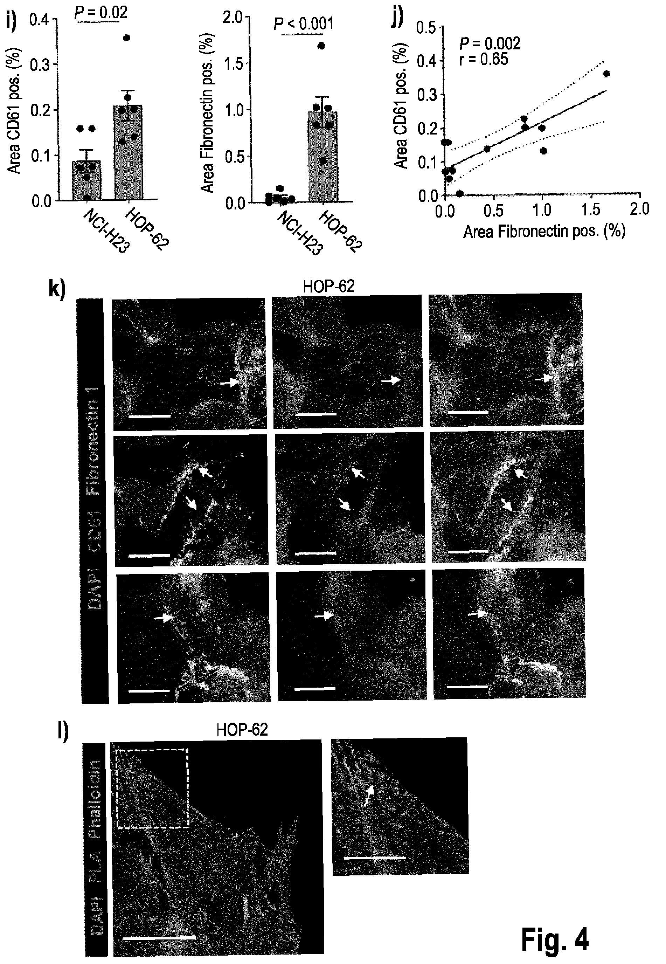

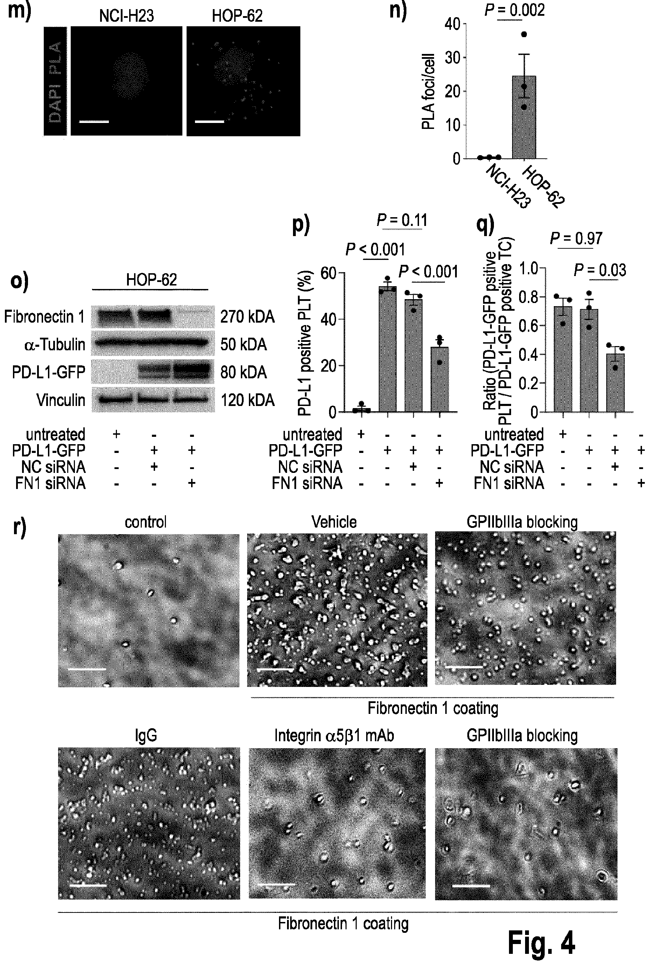

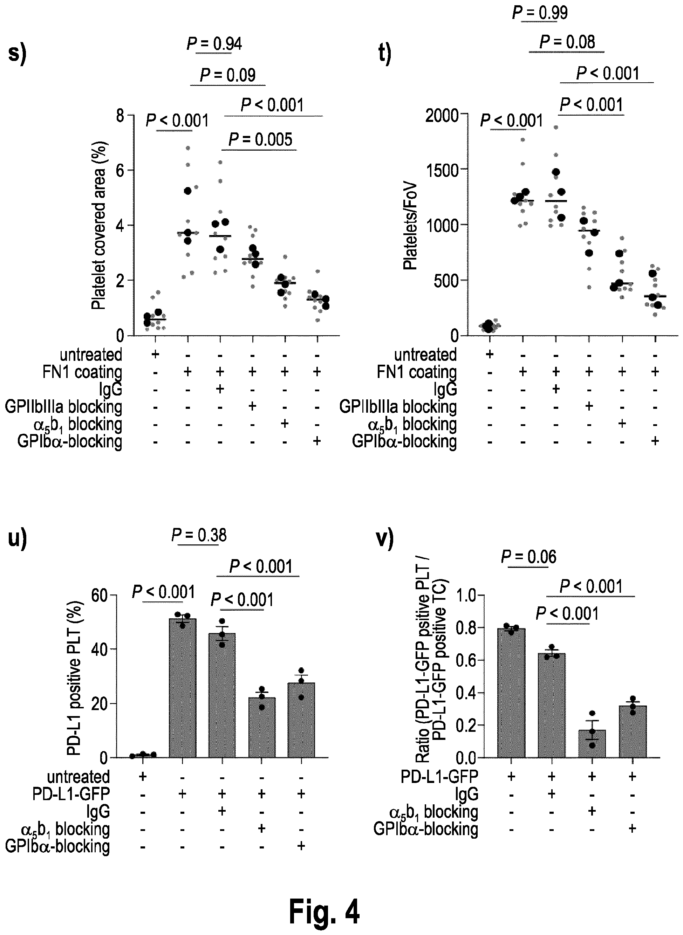

Fig. 4: Fibronectin 1 mediates platelet adhesion to tumor cells and facilitates PD-L1 protein transfer. a-b Expression of GFP (upper) and PD-L1 (lower) on platelet surfaces after co-incubation with FLAG-GFP and PD-L1-GFP in six transfected NSCLC cell lines (A549, NCI-H322, NCI-H522, NCI-H23 and HOP-62, and HOP-92), (n = 3). c-d Ratio of GFP positive platelets / GFP positive tumor cells and PD-L1-GFP positive platelets / PD-L1-GFP positive tumor cells after co-incubation of platelets with transfected NSCLC cell lines (n = 3). Data are mean ± SEM. Statistical significance was calculated by ANOVA and Tukey's multiple comparisons test. e Left, Heat map of relative fibrinogen (FBG), tissue factor (F3), fibronectin 1 (FN1) and von Willebrand factor (VWF) mRNA levels in all tested NSCLC cell lines (n = 3). f Relative mRNA level of FN1 in all tested NSCLC cell lines. Data are mean ± SEM. Statistical significance was calculated by ANOVA and Tukey's multiple comparisons test. g Correlation ratio of PD-L1-GFP positive platelets / PD-L1-GFP positive tumor cells and relative FN1 mRNA level (n = 3). Correlation was determined by simple linear regression analysis. h Immunofluorescence images of platelet adhesion to NCI-H23 and HOP-62 cells (fibronectin is stained in green, platelets in red) (n = 3). Upper scale bar, 100 $\mu$m, lower scale bar, 20 $\mu$m. i Left, Quantification of adhesive platelets after coincubation with NCI-H23 and HOP-62. Quantified as CD61 positive area in % / FoV (n=6 out of 3 independent experiments). Right, quantification of fibronectin covered area in % / FoV in NCI-H23 and HOP-62 cells (n=6 out of 3 independent experiments). Data are mean ± SEM. Statistical significance was calculated by Student's t-test. j Correlation of platelet and fibronectin covered area in % / FoV. Correlation was determined by simple linear regression analysis. k Immunofluorescence images of PD-L1 and fibronectin expression in HOP-62 cells (n = 2). Scale bar 20 $\mu$m. l Representative PLA with PD-L1 and fibronectin in HOP-62 cells. Left scale bar 20 $\mu$m, right scale bar 10 $\mu$m. m Representative PLA with PD-L1 and fibronectin in NCI-H23 and HOP-62 cell (n = 3). Scale bar 10 $\mu$m. n PLA quantification of foci/cell in 119 NCI-H23 and 126 HOP-62 cells out of 3 biological replicates. Data are mean ± SEM. Statistical significance was calculated by Student's t-test. o Western blot analysis for PD-L1 and fibronectin in PD-L1-GFP-transfected HOP-62 cells after siRNA knockdown for fibronectin. Vinculin and $\alpha$-Tubulin was used as loading control. p Expression of PD-L1 on platelets after co-incubation with PD-L1-GFP, PD-L1-GFP/siFN1, and PD-L1-GFP/siNC-transfected HOP-62 cells (n = 3). q Ratio PD-L1-GFP positive platelets / PD-L1-GFP positive tumor cells after co-incubation of platelets with PD-L1-GFP, PD-L1-GFP/siFN1, and PD-L1-GFP/siNC-transfected HOP-62 cells (n = 3). Data are mean ± SEM. Statistical significance was calculated by ANOVA and Tukey's multiple comparisons test. r Representative images of platelet adhesion to fibronectin-coated surface in the presence or absence of different platelet-blocking agents (n = 3). Scale bar 20 $\mu$m. s-t Quantitative analysis of the platelet adhesion assay as platelet covered area / FoV in % (s) and platelets / FoV (t) (n = 9 (small symbols) were analysed out of a total of n = 3 independent experiments (large symbols)). Horizontal line represent mean. Statistical significance was calculated by ANOVA and Tukey's multiple comparisons test. u Quantification of PD-L1 on platelets after co-incubation with PD-L1-GFP transfected HOP-62 cells with or without pre-treatment with platelet-blocking agents (n = 3). v Ratio PD-L1-GFP positive platelets / PD-L1-GFP positive tumor cells after coincubation of platelets with PD-L1-GFP transfected HOP-62 cells with or without pre-treatment with platelet-blocking agents (n = 3). Data are mean ± SEM. Statistical significance was calculated by ANOVA and Tukey's multiple comparisons test.

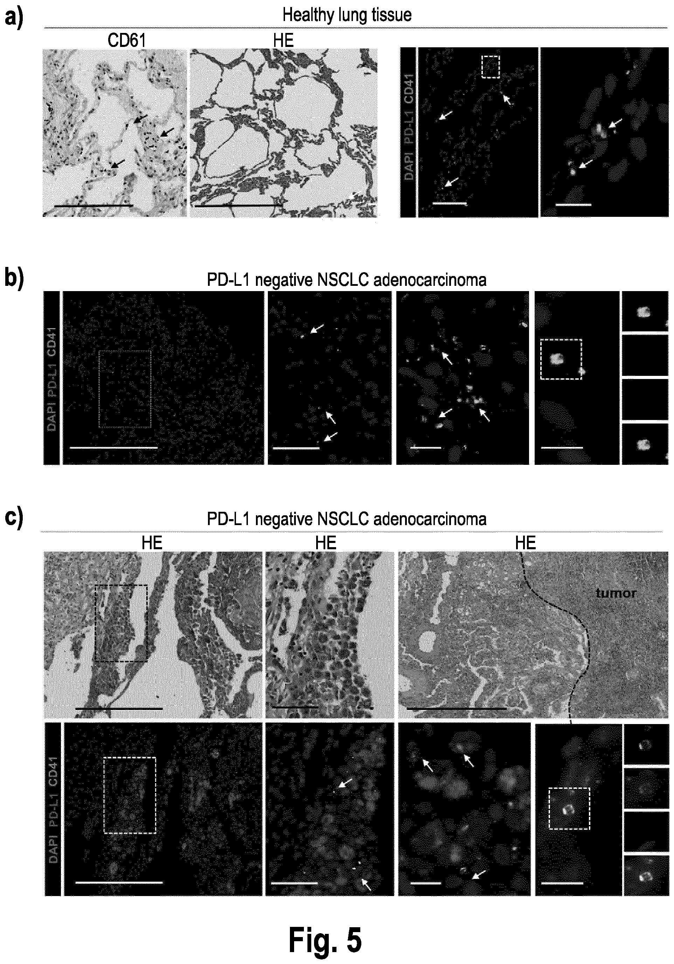

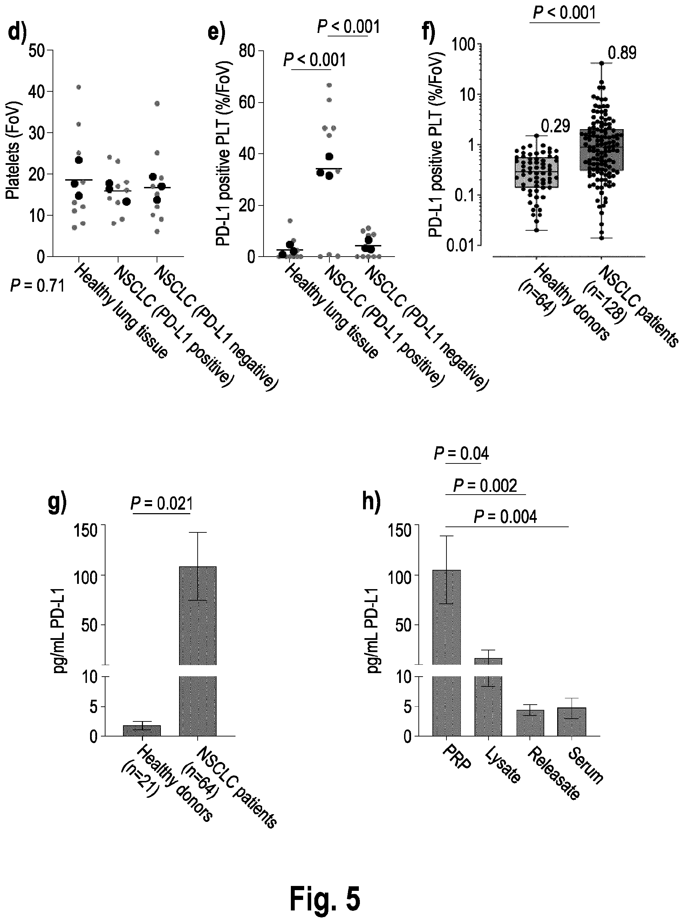

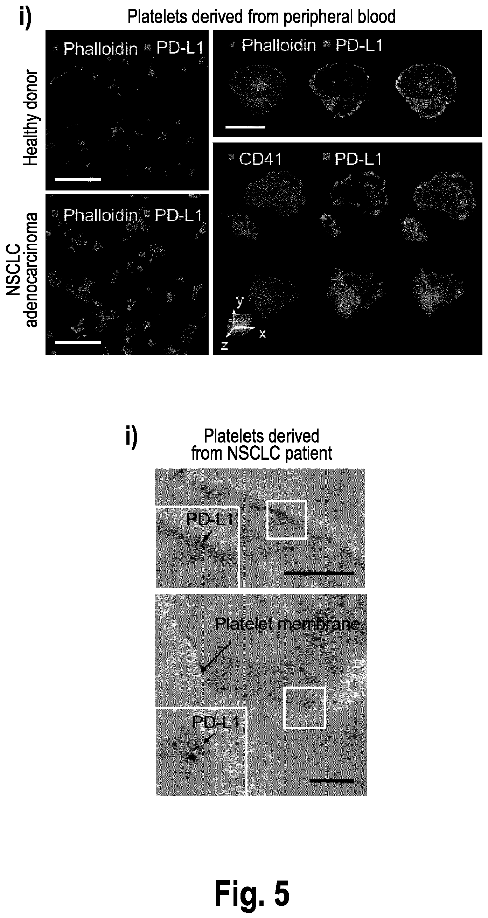

Fig. 5: Platelets from non-small lung cancer (NSCLC) patients show increased PD-L1 levels. a Left panel, left, Immunohistochemistry for CD61 in healthy human lung tissue (black arrow highlights CD61 pos. platelets). Scale bar 200 $\mu$m. Left panel, right, Representative micrograph of healthy lung tissue (H&E staining). Scale bar 500 $\mu$m. Right panel, Immunofluorescence microscopy for CD41 (green) positive, PD-L1 negative platelets in healthy lung tissue. Nuclei stained with DAPI (n = 3). Left scale bar 100 $\mu$m, right scale bar 10 $\mu$m. b Immunofluorescence staining for CD41 (green) and PD-L1 (red) on platelets in a PD-L1 negative NSCLC patient tumor sample; nuclei stained with DAPI (n = 3). Left scale bar 500 $\mu$m, center left scale bar 50 $\mu$m, center right and right scale bar 10 $\mu$m. c Upper, Representative mi-crographs of NSCLC adenocarcinoma (H&E staining) (n = 3). Left scale bar 250 $\mu$m, center scale bar 50 $\mu$m, right scale bar 500 $\mu$m. Lower, Immunofluorescence staining for CD41 (green) and PD-L1 (red) (n = 3). Left scale bar 500 $\mu$m, center left scale bar 50 $\mu$m, center right and right scale bar 10 $\mu$m. d Quantitative analysis of the platelets / FoV in healthy lung tissue (n = 3), PD-L1 positive (n = 3) and PD-L1 negative (n = 3) NSCLC patients. (n = 9 (small symbols) were analysed out of a total of n=3 independent experiments (large symbols). e Quantitative analysis of PD-L1 positive platelets (%/ FoV) in healthy lung tissue (n = 3), PD-L1 positive (n = 3) and PD-L1 negative (n = 3) NSCLC patients. d-e, Horizontal line represent mean. Statistical significance was calculated by ANOVA and Tukey's multiple comparisons test. f Percentage of PD-L1-positive platelets in 64 healthy donors and 128 NSCLC patients. Each dot represents a single donor. Data are median and IQR. Statistical significance was calculated by Mann Whitney test. g Total amount of PD-L1 (pg/mL) in healthy donors (n = 21) and NSCLC patients (n = 64) analysed by ELISA. Protein level were analyzed in 64 out of 128, randomly assigned patients of the NSCLC cohort. Data are mean ± SEM. Statistical significance was calculated by Mann Whitney test. h Total amount of PD-L1 quantity (pg/mL) in PRP, platelet lysate, platelet releasate, and serum (n = 6). Data are mean ± SEM. Statistical significance was calculated by ANOVA and Tukey's multiple comparisons test. i Upper left, Representative PD-L1 immunofluorescence staining of healthy donor platelets. Lower left, PD-L1 expression on platelets of a NSCLC patient. Platelets were counter stained with phalloidin. Upper right, Expression pattern of PD-L1 on a platelet derived from a NSCLC patient (counter stained with phalloidin). Lower right, PD-L1 expression on a platelet of a NSCLC patient counter stained with CD41. Left scale bar 10 $\mu$m, right scale bar 2 $\mu$m. j Left, platelets of a NSCLC patient, assessed by transmission electron microscopy. PD-L1 stained with post-embedding immunogold labelling. Upper right and lower right, PD-L1 gold particles densely accumulating on the platelet membrane (black dots). Left scale bar 2 $\mu$m, right scale bar 100 nm.

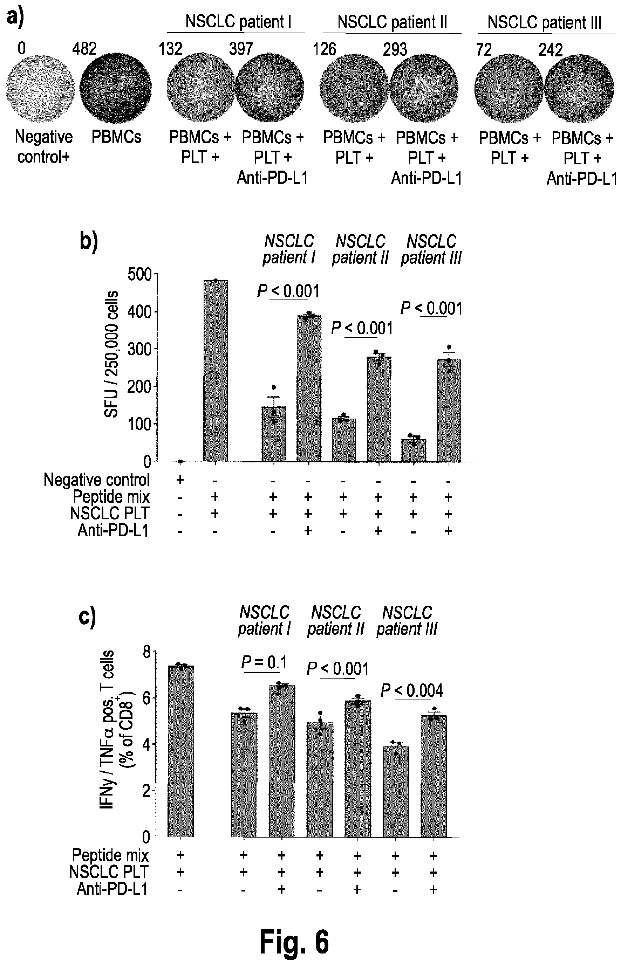

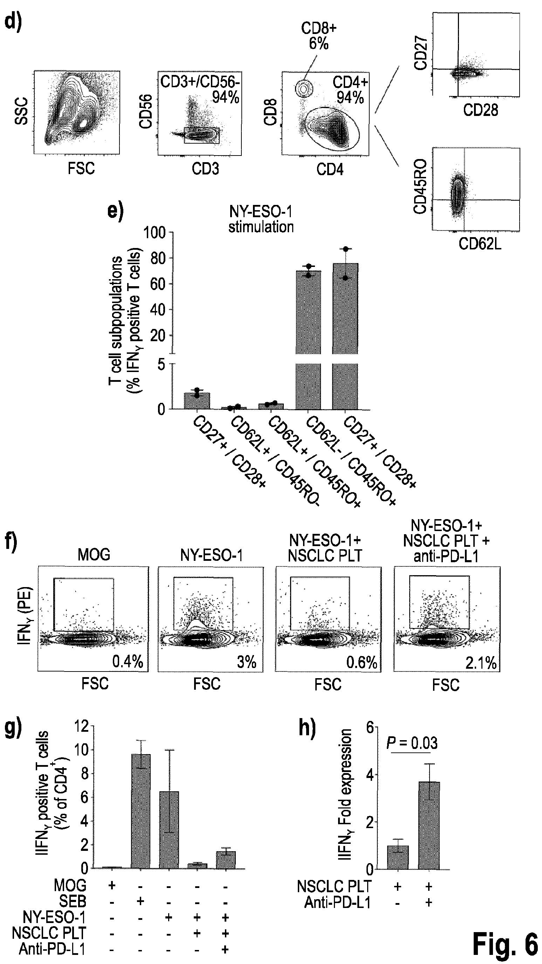

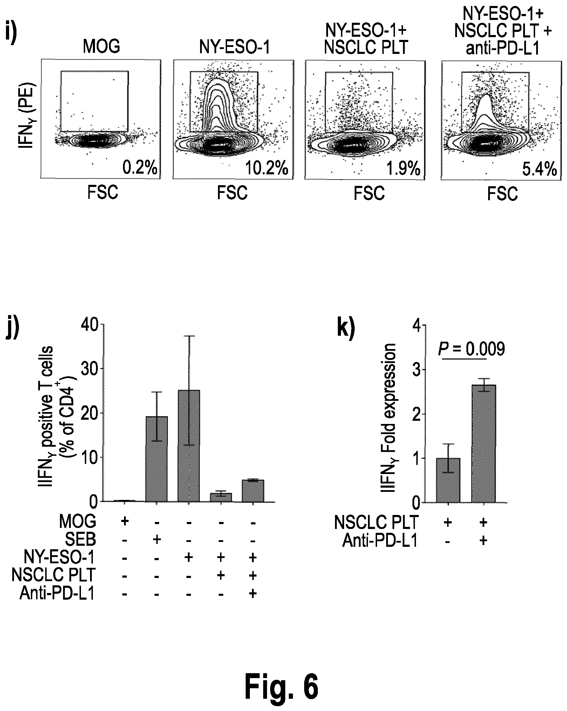

Fig. 6: PD-L1 on platelets shows functional relevance via decreasing T-cell activity. a IFN$\gamma$ ELISPOT assay of peptide-specific T-cells co-incubated with PD-L1 positive platelets with or without anti-PD-L1 mAb pre-treatment (n = 3). b Quantification of the IFN$\gamma$ ELISPOT assays (n = 3). c Flow cytometry-based quantification of indicated cytokines and surface markers for peptide stimulated CD8+ T-cells co-incubated with PD-L1 positive platelets with or without anti-PD-L1 mAb pre-treatment (n = 3). b-c Data are mean ± SEM. Statistical significance was calculated by Student's t-test. d Representative fluorescence-activated cell sorting plots showing the gating strategy and the T-cell subpopulations after pre-sensitization, enrichment, and expansion. e Quantitative sub-phenotyping of NY-ESO-1 specific T-cells using flow cytometry (n = 2). Data are mean ± SEM. f Representative plots displaying CD4$^+$ TEM activity levels measured by INF$\gamma$ expression after co-incubation with PD-L1 positive platelets with or without anti-PD-L1 mAb pre-treatment (n = 3). g Quantification of INF$\gamma$ positive CD4$^+$ TEM. h IFN$\gamma$ fold change in CD4$^+$ TEM (n = 3). i Representative plots displaying CD4$^+$ TEM activity levels measured by TNF$\alpha$ expression after co-incubation with PD-L1 positive platelets with or without anti-PD-L1 mAb pre-treatment (n = 3). j Quantification of TNF$\alpha$ positive CD4$^+$ TEM. k, TNF$\alpha$ fold change in CD4$^+$ TEM (n = 3). g-h, j-k Data are mean ± SEM. Statistical significance was calculated by Student's t-test.

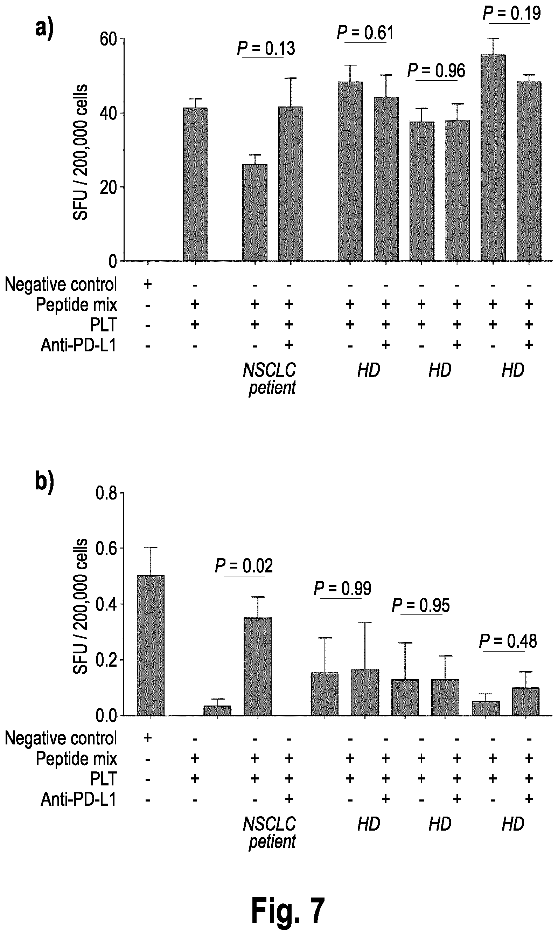

Fig. 7: Immunomodulation of platelets derived from healthy donors. a Quantification of the IFN$\gamma$ ELISPOT assays in three healthy donors and one NSCLC patient. b Flow cytometry-based quantification of indicated cytokines and surface markers for peptide stimulated CD8$^+$ T-cells co-incubated with PD-L1 positive platelets with or without anti-PD-L1 mAb pre-treatment (n = 4). b-c Data are mean ± SEM. Statistical significance was calculated by Student's t-test.

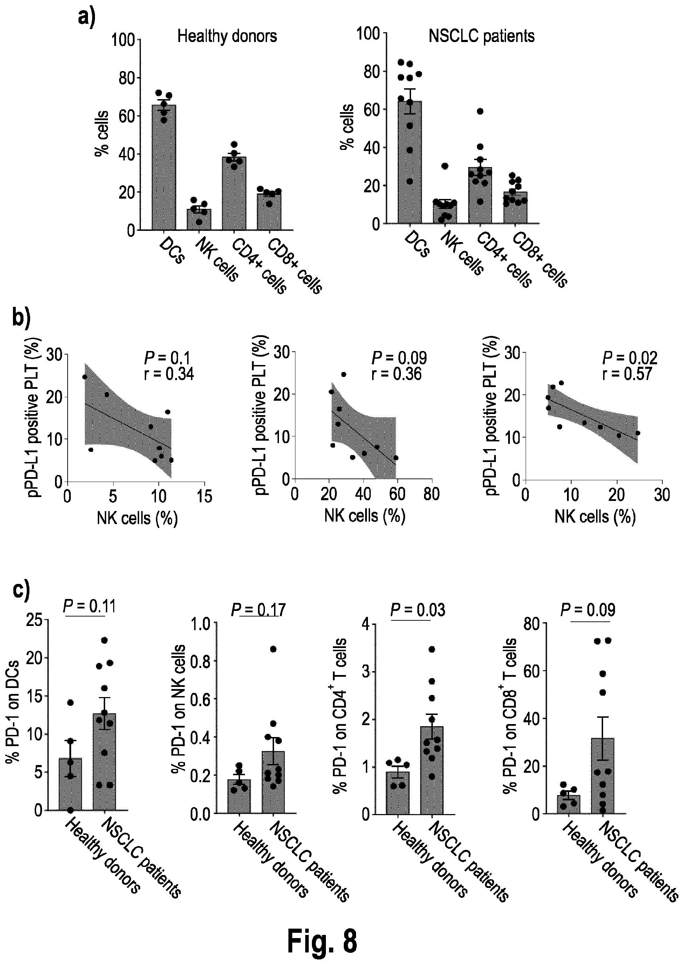

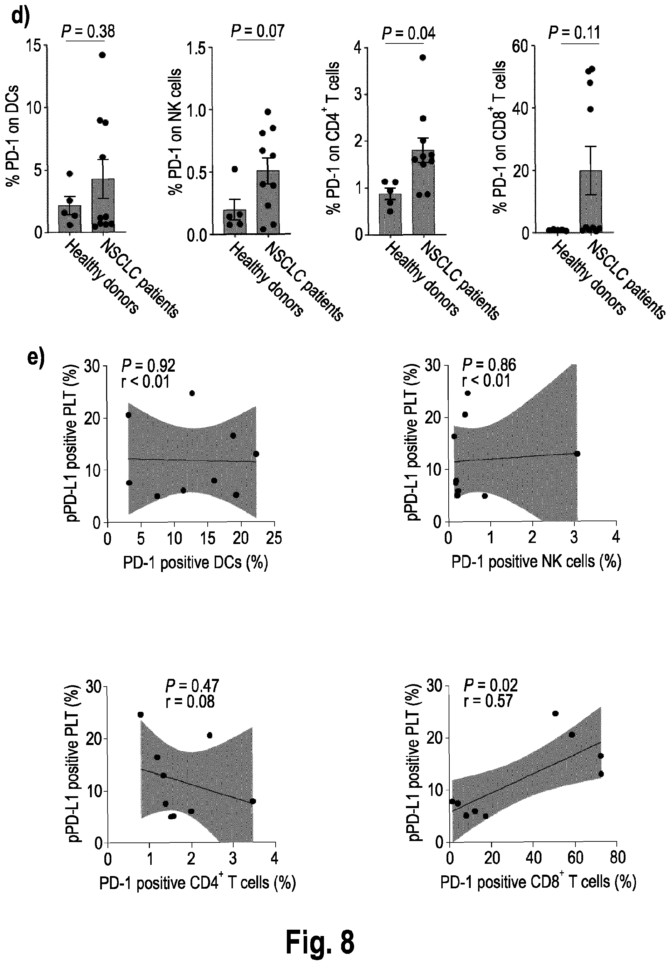

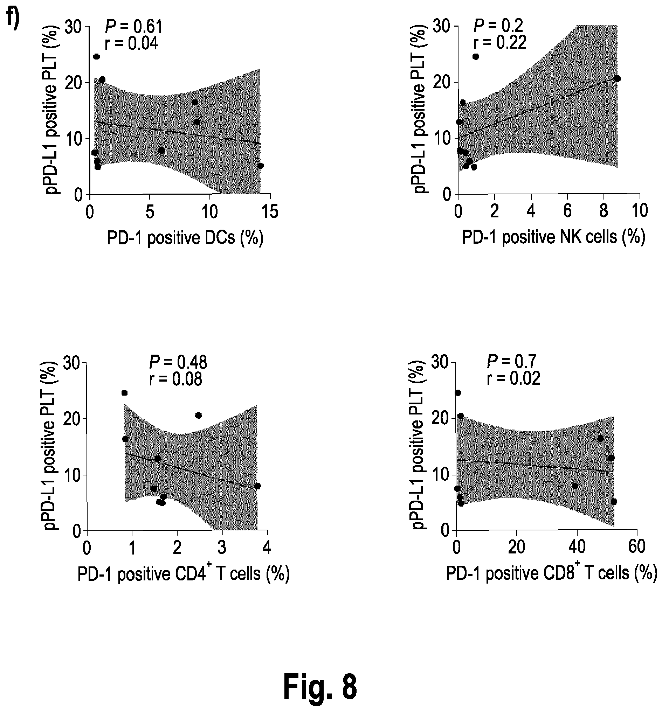

Fig. 8: Immunophenotyping in NSCLC patients. a Flow cytometry-based determination of immune cell distribution in the peripheral blood of healthy donors (n = 5) and NSCLC patients (n = 10). Data are mean ± SEM. b Correlation of pPD-L1 level and total number of NK, CD4$^+$ and CD8$^+$ cells. Correlation was determined by simple linear regression analysis. c Analysis of PD-1 expression on DCs, NK cells CD4$^+$ and CD8$^+$ cells in healthy donors and NSCLC patients. Data are mean ± SEM. Statistical significance was calculated by Student's t-test and Mann-Whitney test. d Analysis of PD-L1 expression (%) on DCs, NK cells CD4$^+$ and CD8$^+$ cells in healthy donors and NSCLC patients. Data are mean ± SEM. Statistical significance was calculated by Student's t-test and Mann-Whitney test. e Correlation of pPD-L1 level and number of PD-1 positive NK, CD4$^+$ and CD8$^+$ cells. Correlation was determined by simple linear regression analysis. f Correlation of pPD-L1 level and number of PD-L1 positive NK, CD4$^+$ and CD8$^+$ cells. Correlation was determined by simple linear regression analysis.

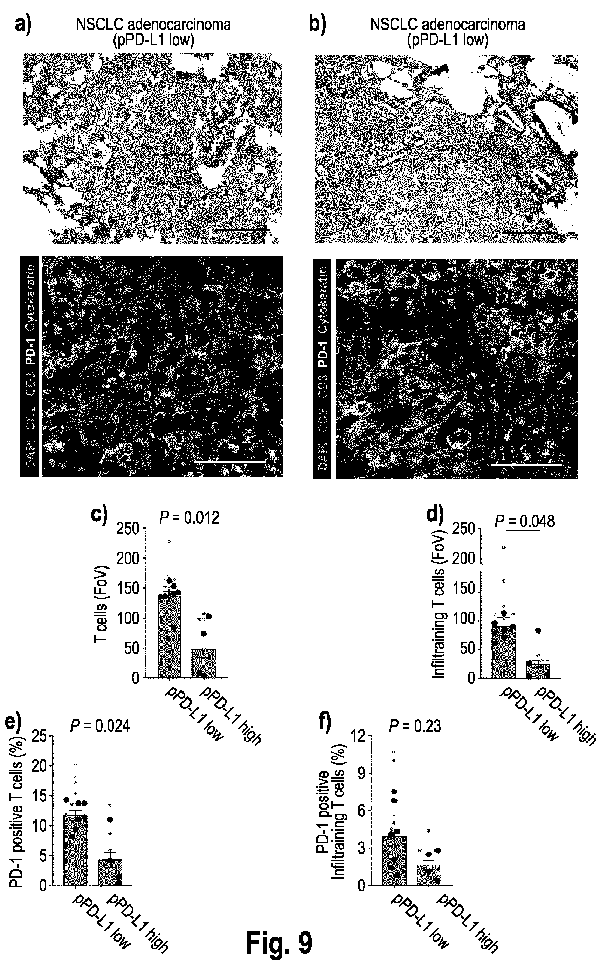

Fig. 9: pPD-L1 correlates with T cell infiltration in NSCLC. a-b Upper, Representative micrographs of NSCLC adenocarcinoma (H&E staining) (n = 11). Left scale bar 500 $\mu$m. Immunofluorescence staining for T cells (CD2, CD3 and PD-1) in the TME of a NSCLC patient presenting with high pPD-L1 (a) and low pPD-L1 (b). Each image is representative for at least two regions of interest (ROI) in each tumor sample. ROI were selected based on manual prestaining of DAPI. Scale bar= 100 $\mu$m, (n = 11). c Quantification of T cells per FoV. pPD-L1 high vs. low was defined according to the median expression in this cohort. d Quantification of PD-1 positive T cells per FoV (%). e Quantification of infiltrating T cells per FoV. f Quantification of PD-1 positive infiltrating T cells per FoV (%), c-f A total number of n = 22 ROIs (small symbols) were analysed out of a total of n = 11 patients (large symbols). Data are mean ± SEM. Statistical significance was calculated by Student's t-test or Mann Whitney test.

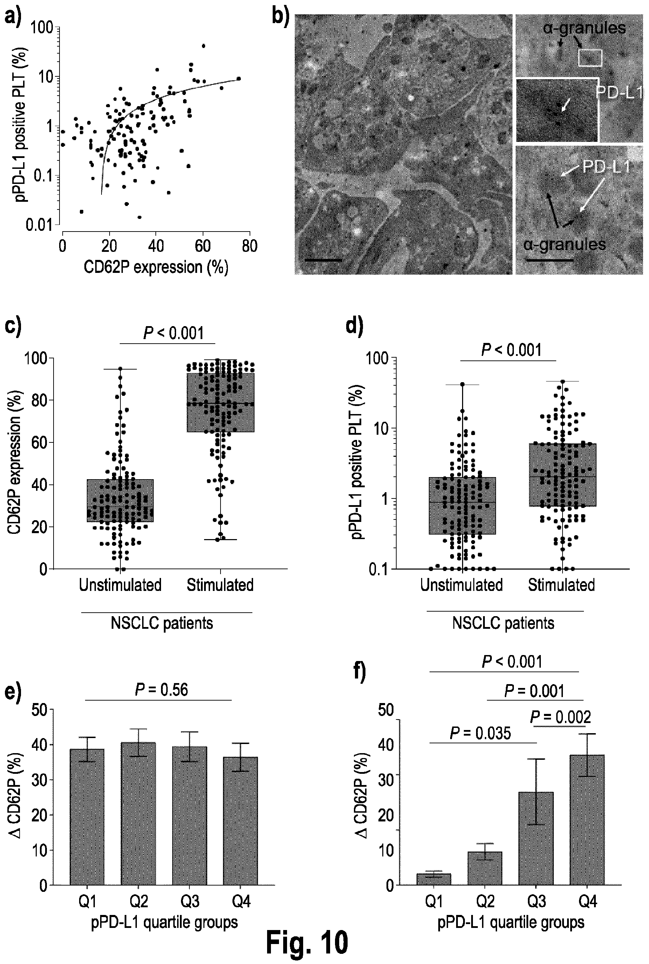

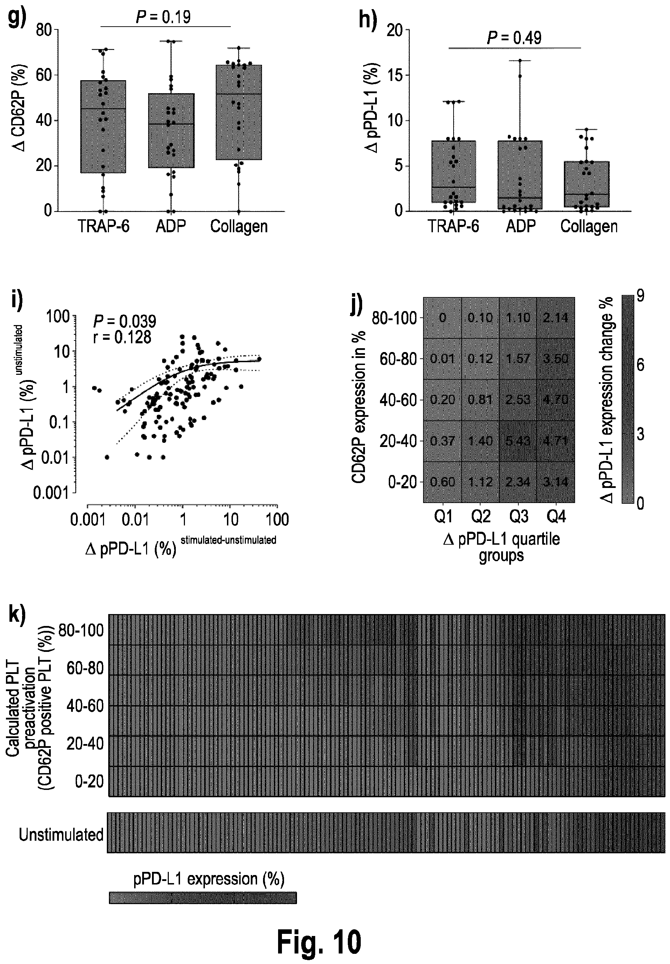

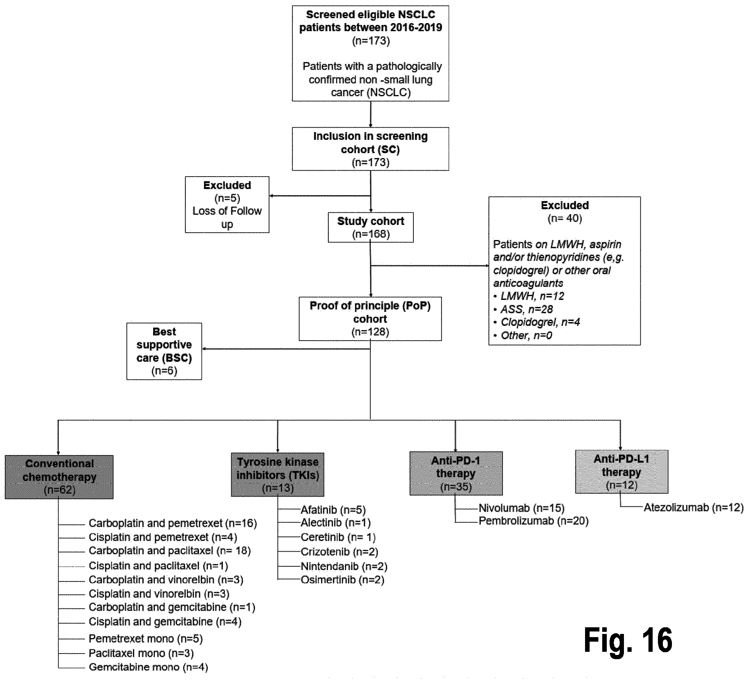

Fig. 10: Platelets from NSCLC patients show increased PD-L1 protein levels upon activation. a Correlation between platelet-derived PD-L1 (pPD-L1) and platelet activation (CD62P expression) in 128 NSCLC patients. Each dot represents a single patient. Correlation was determined by simple linear regression analysis. b Platelets from a NSCLC patient assessed by transmission electron microscopy. PD-L1 stained with post-embedding immunogold labelling. Upper right and lower right, PD-L1 gold particles densely accumulate in $\alpha$-granules. Left scale bar 1 $\mu$m, right scale bar 100 nm. c-d, Changes in CD62P (c) and the PD-L1 (d) levels upon platelet stimulation with 10$\mu$M TRAP-6 for 2 minutes in 128 NSCLC patient samples. Data are median and IQR. Statistical significance was calculated by Mann Whitney test. e CD62 expression change $\Delta$CD62P (CD62P in stimulated platelets in %-CD62P in unstimulated platelets in %) after platelet activation with 10$\mu$M TRAP-6 (n=24), 2.5 $\mu$M ADP (n=24), or 5$\mu$g/ml collagen (n=24). f PD-L1 expression change $\Delta$PD-L1 (PD-L1 in stimulated platelets in % - PD-L1 in unstimulated platelets in %) in NSCLC patients after platelet activation. Data are median and IQR. Statistical significance was calculated by KruskalWallis test. g CD62 expression change ($\Delta$CD62P) in the different pPD-L1 quartile groups identified in unstimulated platelets of NSCLC patients (n = 128). h PD-L1 expression change ($\Delta$PD-L1) in the different pPD-L1 quartile groups identified in unstimulated platelets of NSCLC patients (n =128). Data are mean ± SEM. Statistical significance was calculated by Friedman and Dunn's multiple comparisons test. i Correlation between pPD-L1 expression in unstimulated platelets of 128 NSCLC patients and the $\Delta$PD-L1 upon platelet stimulation with 10$\mu$M TRAP-6. Each dot represents a single patient. Correlation was determined by simple linear regression analysis. j Heatmap shows the calculated $\Delta$PD-L1 depending on the CD62P activation ranges (y-axis) and the quartiles of pPD-L1 level (x-axis) calculated in pooled data from 128 NSCLC patients. For details of subsampling and calculation used, see Methods and Fig. 16. k Adjusted pPD-L1 levels in all 128 NSCLC patients upon calculated platelet pre-activation ranges (by CD62P expression level).

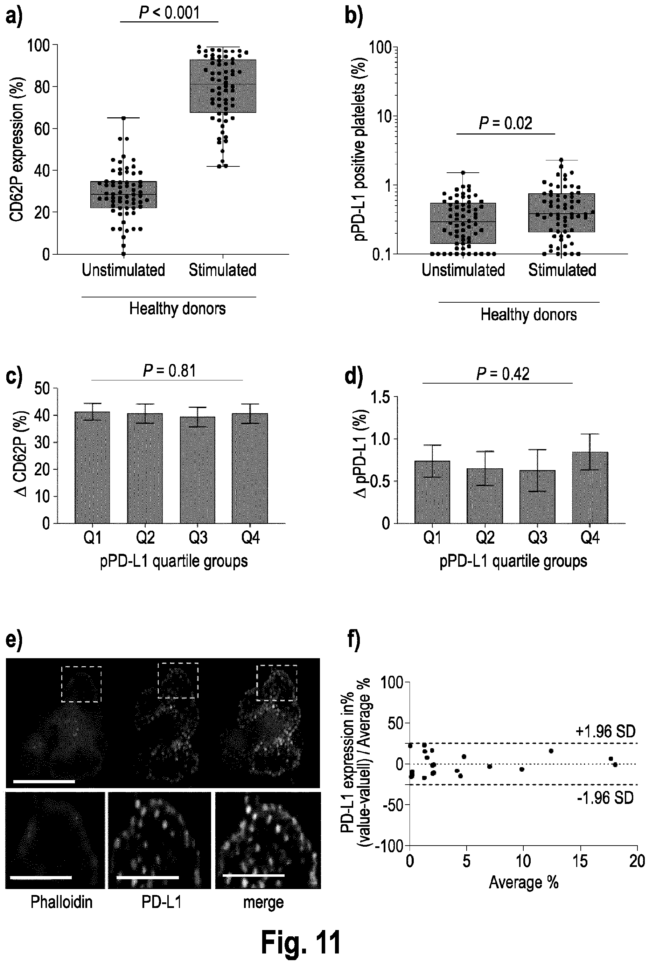

Fig. 11: Association of pPD-L1 expression and platelet activation in healthy donors. a-b Changes in CD62P (a) and the PD-L1 (b) levels upon platelet stimulation with 10$\mu$M TRAP-6 for 2 minutes in 64 healthy donors. Data are median and IQR. Statistical significance was calculated by Mann Whitney test. c CD62 expression change ($\Delta$CD62P) in the different pPD-L1 quartile groups identified in unstimulated platelets of healthy donors (n = 64). d PD-L1 expression change ($\Delta$PD-L1) in the different pPD-L1 quartile groups identified in unstimulated platelets of healthy donors (n = 64). Data are mean ± SEM. Statistical significance was calculated by Friedman and Dunn's multiple comparisons test. e Representative PD-L1 immunofluorescent staining on platelets derived from a NSCLC patient (TP123). Lower section shows a detailed expression pattern the PD-L1 on platelets. Platelets were counter stained with phalloidin. Up, scale bar 2 $\mu$m, lower scale bar 0.5 $\mu$m. f Bland-Altman plot for the flow cytometry-based determination of PD-L1 on the platelet surface (big dotted line displays: -1.96SD: -25.17, +1.96SD: 25.5, Bias: 0.17) (n = 21).

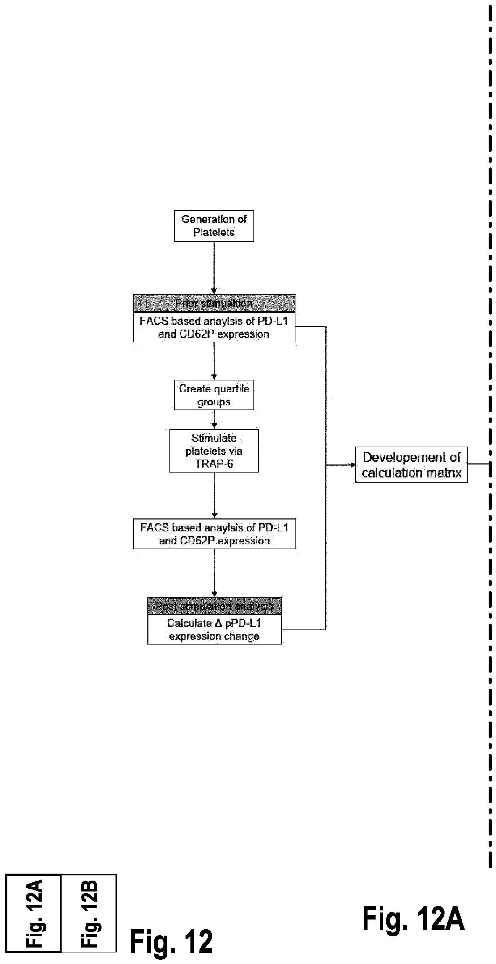

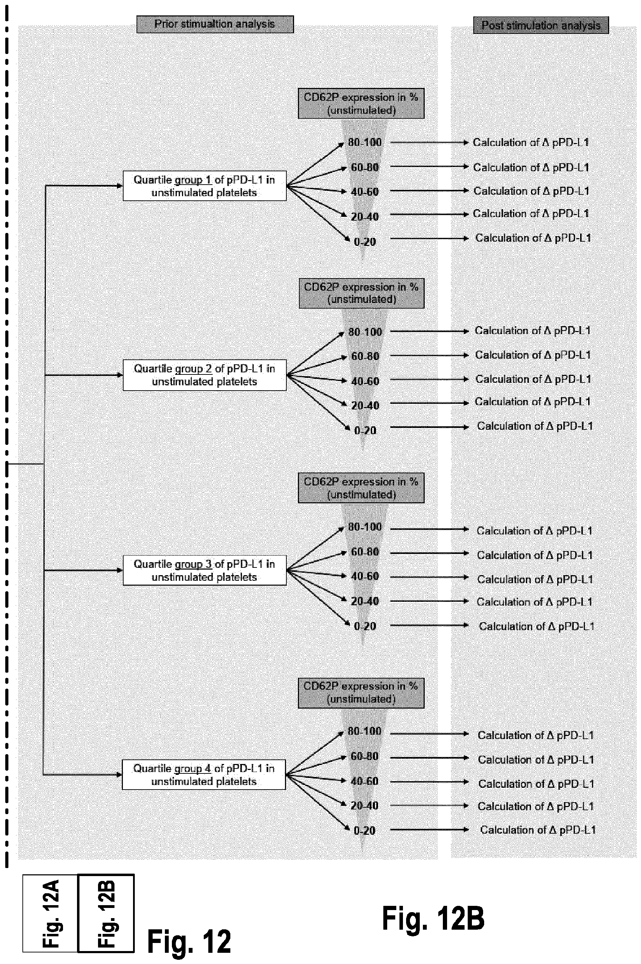

Fig. 12: Algorithm of pPD-L1$^{adj.}$ calculation. Flow chart presenting the establishment of an activation-independent calculation matrix for platelet PD-L1.

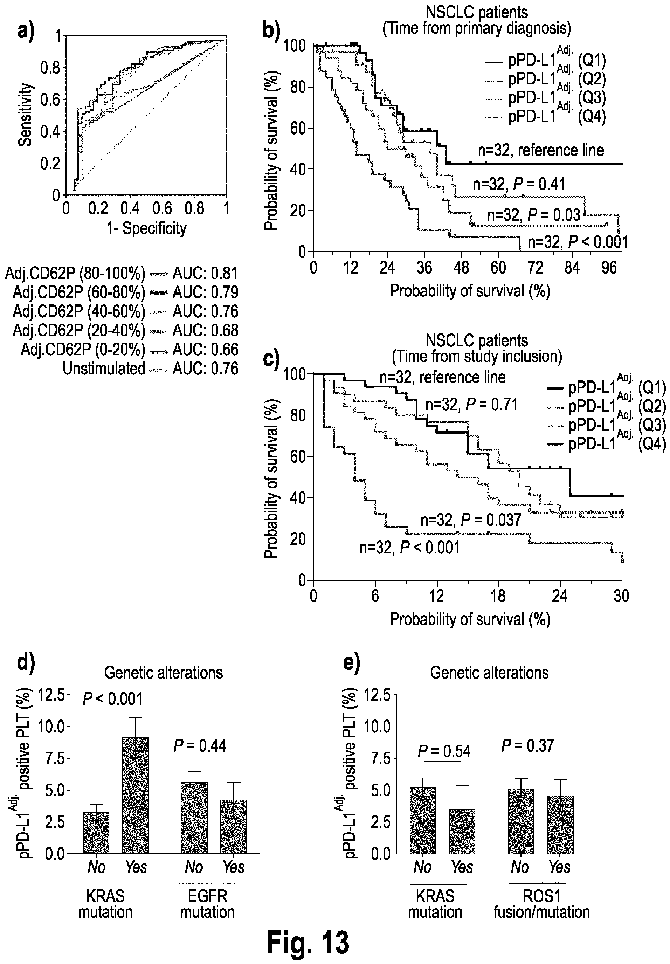

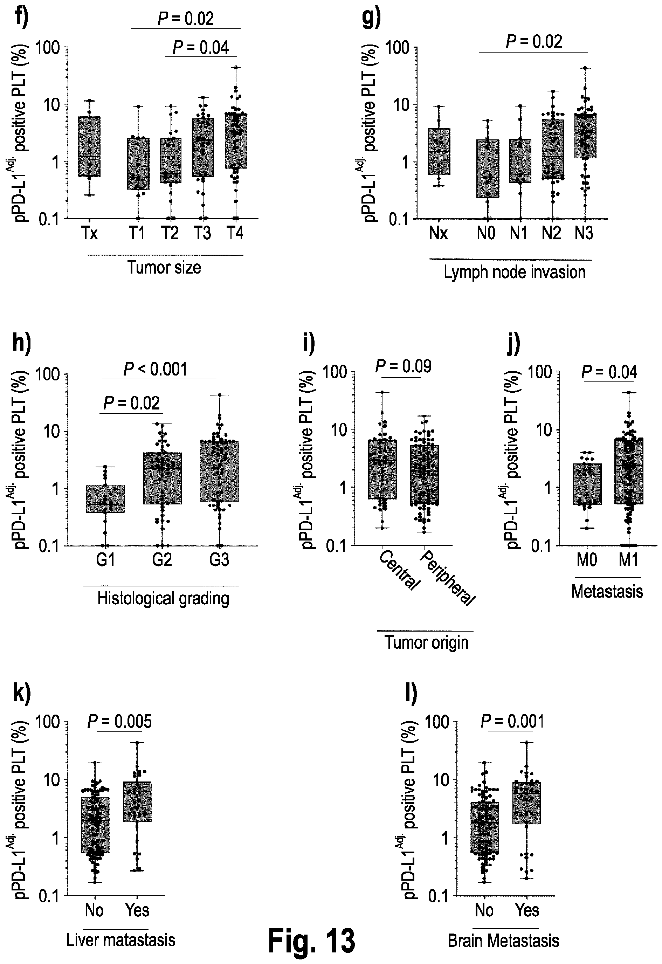

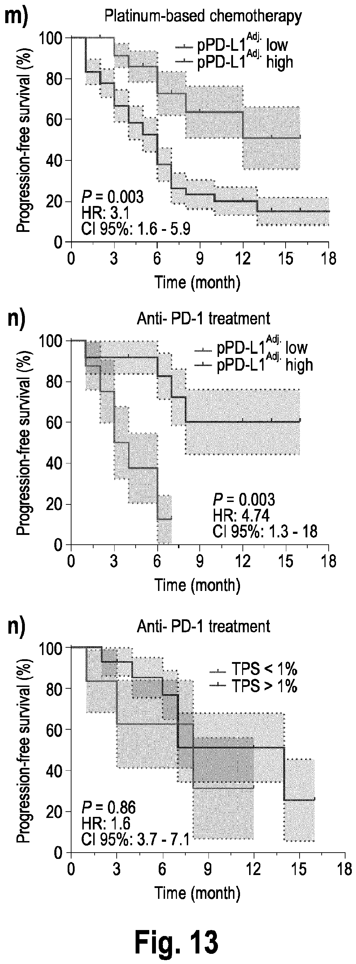

Fig. 13: Platelet-derived PD-L1 as a novel prognostic and predictive marker in NSCLC a Combined estimate of platelet CD62P (0-100%) and pPD-L1 levels predicts overall survival (OS) in 128 NSCLC patients; receiver-operating characteristics (ROC) analysis. b Kaplan-Meier analysis of overall survival (OS) dependent on the pPD-L1$^{adj.}$ as defined by quartile groups (very low (Q1), low (Q2), high (Q3) and very high (Q4)). Survival data refer to the time point of primary diagnosis (n = 128). c Overall survival (OS) in different pPD-L1$^{adj.}$ quartile groups according to the time point of platelet analysis (n = 128). b-c Statistical significance was calculated by log-rank test. d-e Association of pPD-L1$^{adj.}$ levels and different genetic alterations (KRAS (n=38), EGFR (n=19), EML-4-ALK (n=5) and ROS-1 (n=6)) (wt = wild type, mut = mutation). Data are mean ± SEM. Statistical significance was calculated by Mann-Whitney test. f-h pPD-L1Adj levels in patients with different tumor stage (T1-4), lymph node invasion (NO-3), and grade (G1-3) (n = 128). Each dot represents a single patient. Data are median and IQR. Statistical significance was calculated by Friedman and Dunn's multiple comparisons test. i pPD-L1$^{adj.}$ levels are associated with tumor origin (n = 128). j-l pPD-L1$^{adj.}$ is associated with the occurrence of metastasis (in general and at specific sites, including liver and brain) (n = 128). f-l Each dot represents a single patient. Data are median and IQR. Statistical significance was calculated by Mann-Whitney test. m Kaplan-Meier curves estimates of PFS in patients with a pPD-L1Adj level > median (red) and pPD-L1$^{adj.}$ level < median (blue) treated with conventional chemotherapy (n = 62). n Kaplan-Meier curves estimates of PFS in patients with a pPD-L1$^{adj.}$ level > median (red) and pPD-L1$^{adj.}$ level < median (blue) treated with anti-PD-1 therapy (n = 20). o Kaplan-Meier curves estimates of PFS in patients with a TPS score > 1% (red) and TPS < 1% (blue) treated with anti-PD-1 therapy (n = 20). m-o Statistical significance was calculated by log-rank test.

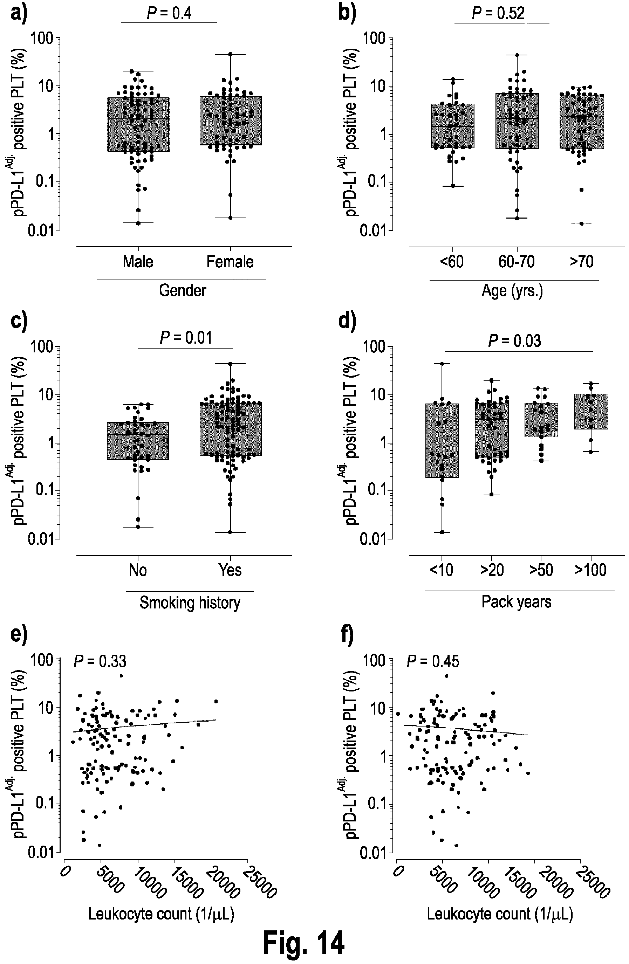

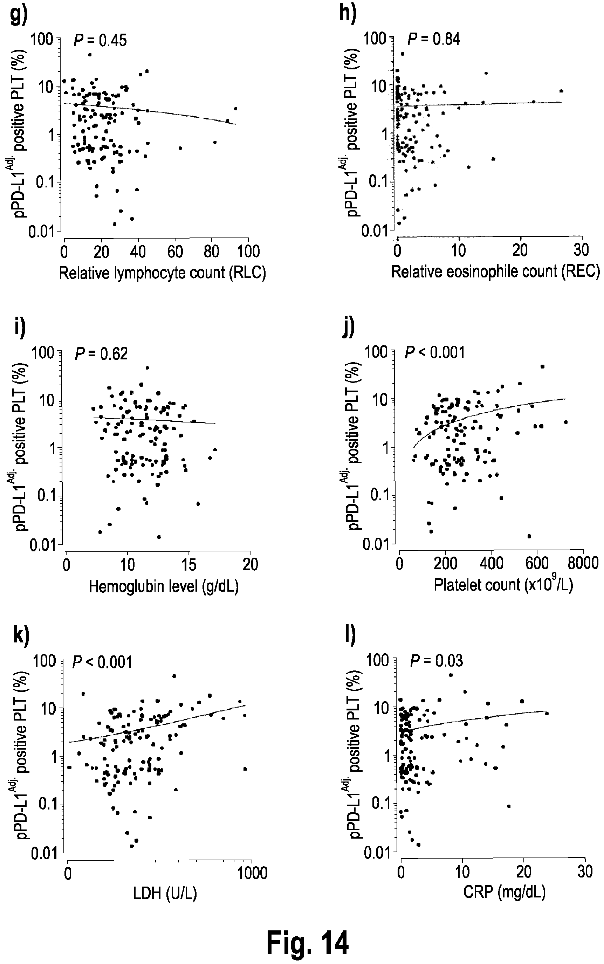

Fig. 14: Correlation of pPD-L1$^{adj.}$ and clinical parameters. a Correlation of pPD-L1$^{adj.}$ levels and gender (n = 128). Each dot represents a single patient. Data are median and IQR. Statistical significance was calculated by Mann-Whitney test. b Correlation of pPD-L1$^{adj.}$ levels and age (n = 128). Each dot represents a single patient. Data are median and IQR. Statistical significance was calculated by Kruskal-Wallis test. c Correlation of pPD-L1$^{adj.}$ levels and smoking history (n = 128). Each dot represents a single patient. Data are median and IQR. Statistical significance was calculated by Mann-Whitney test. d Correlation of pPD-L1$^{adj.}$ levels and pack years (n = 128). Each dot represents a single patient. Data are median and IQR. Statistical significance was calculated by Kruskal-Wallis test. Correlation of pPD-L1$^{adj.}$ levels and leukocyte count (e), lymphocyte count (f), relative lymphocyte count (g), relative eosinophil count (h), haemoglobin level (i), platelet count (j), LDH (k) and CRP (I). Each dot represents a single patient. Correlation was determined by simple linear regression analysis.

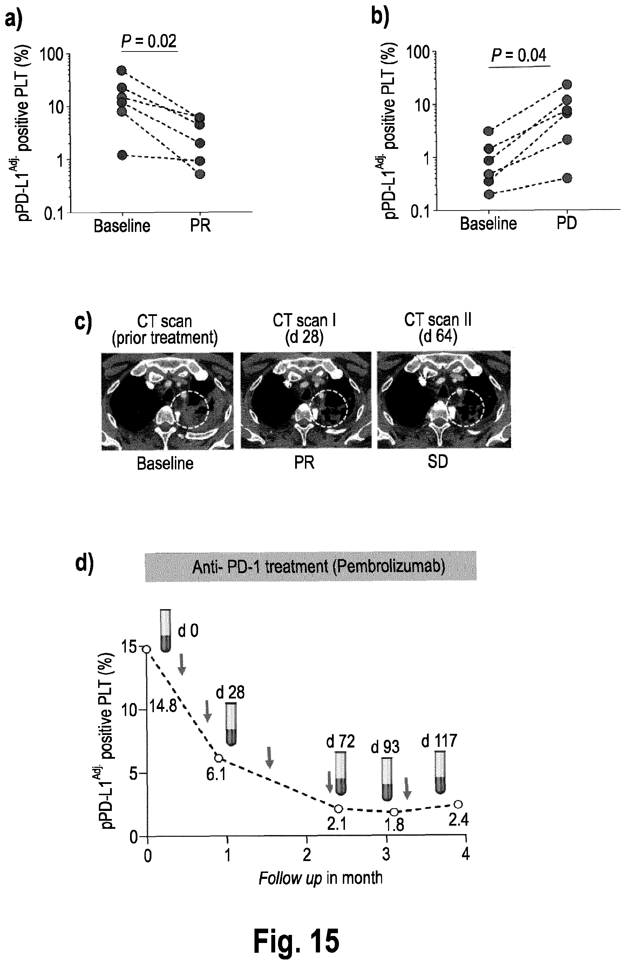

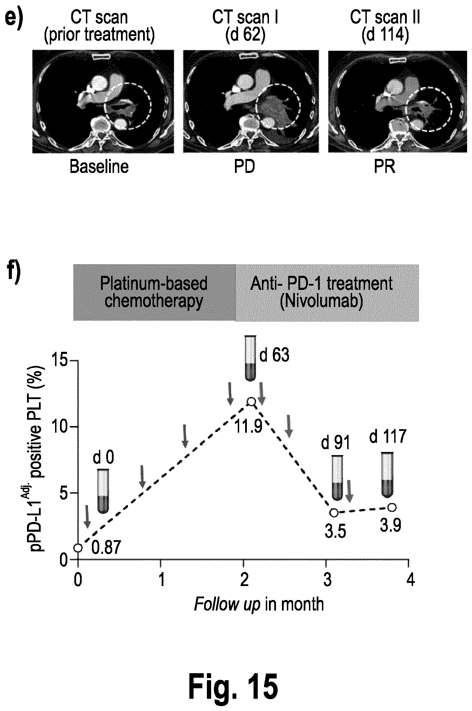

Fig. 15: pPD-L1 predicts treatment response in NSCLC. a-b pPD-L1$^{adj.}$ levels differ in patients presenting with a partial remission (PR) or a progressive disease (PD) (n = 6). Purple dots represent anti-PD-1 treatment, red dots platinumbased chemotherapy. Statistical significance was calculated by Mann-Whitney test. c-f Long-term pPD-L1$^{adj.}$ profiles of two representative NSCLC patients (arrow represents treatment, dots represent determination of pPD-L1$^{adj.}$).

Fig. 16: Flow chart of patient selection. During 2016-2019, 173 patients were screened for eligibility. 173 patients were included in the screening cohort (SC). 40 patients talking anticoagulating agents were excluded from further analysis. 128 patients were finally included in the proof of principle cohort (PoP).

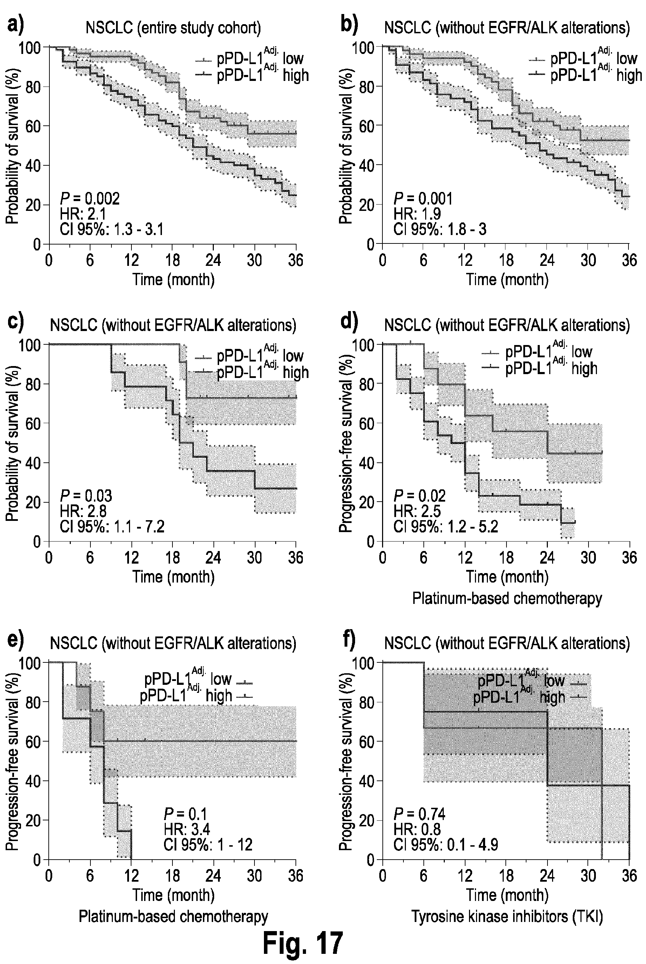

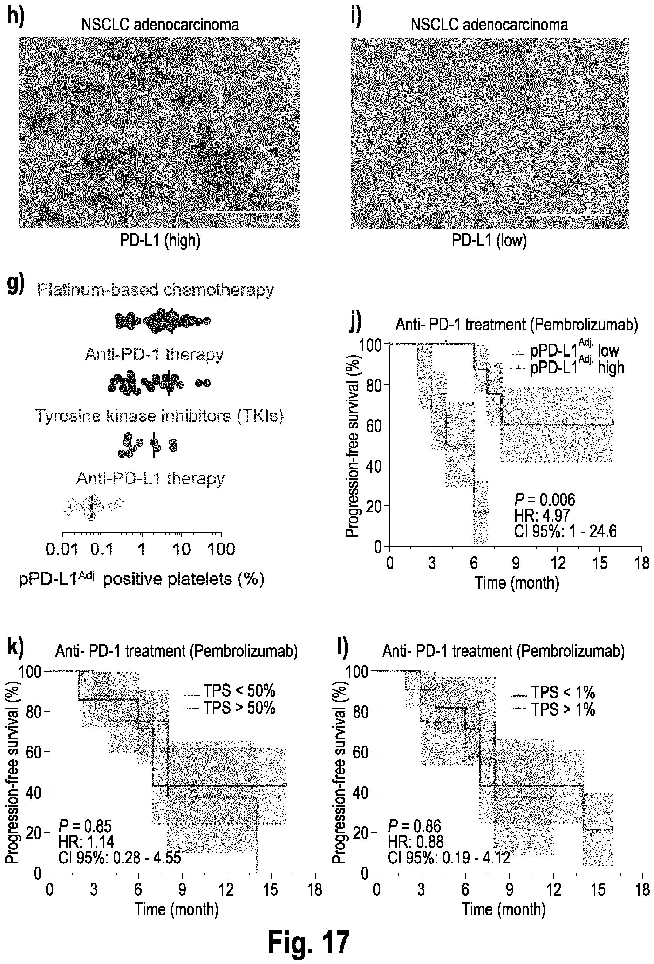

Fig. 17: pPD-L1 as prognostic and predictive marker in NSCLC. a Kaplan-Meier curves estimates of OS in the entire study cohort (n = 128). b-c Kaplan-Meier curves estimates of OS in NSCLC patients with (b) or without EGFR/ALK alterations (c). d Kaplan-Meier curves estimates of PFS in NSCLC patients without EGFR/ALK alterations receiving platinum-based chemotherapy (n = 62). e Kaplan-Meier curves estimates of PFS in NSCLC patients with EGFR/ALK alterations receiving platinum-based chemotherapy (n = 18). f Kaplan-Meier curves estimates of PFS in NSCLC patients with EGFR/ALK alterations receiving tyrosine kinase inhibitors (TKI) (n = 7). a-f pPD-L1$^{adj.}$ level > median (red) and pPD-L1$^{adj.}$ level < median (blue). Statistical significance was calculated by log-rank test. g pPD-L1$^{adj.}$ expression regarding to the treatment regimens. h-i Representative immunohistochemistry showing PD-L1 expression in two NSCLC patients. Scale bar 250 $\mu$m. j Kaplan-Meier curves estimates of PFS in NSCLC patients receiving the anti-PD-1 treatment (Pembrolizumab) (n = 15). pPD-L1$^{adj.}$ level > median (red) and pPD-L1$^{adj.}$ level < median (blue). k-l Kaplan-Meier curves estimates of PFS in NSCLC patients receiving the anti-PD-1 treatment (Pembrolizumab) (n = 15). TPS >50% (k) and TPS>1% (l) is given in red, TPS< 50% (k) and TPS < 1% (l) is given in blue. Statistical significance was calculated by logrank test.

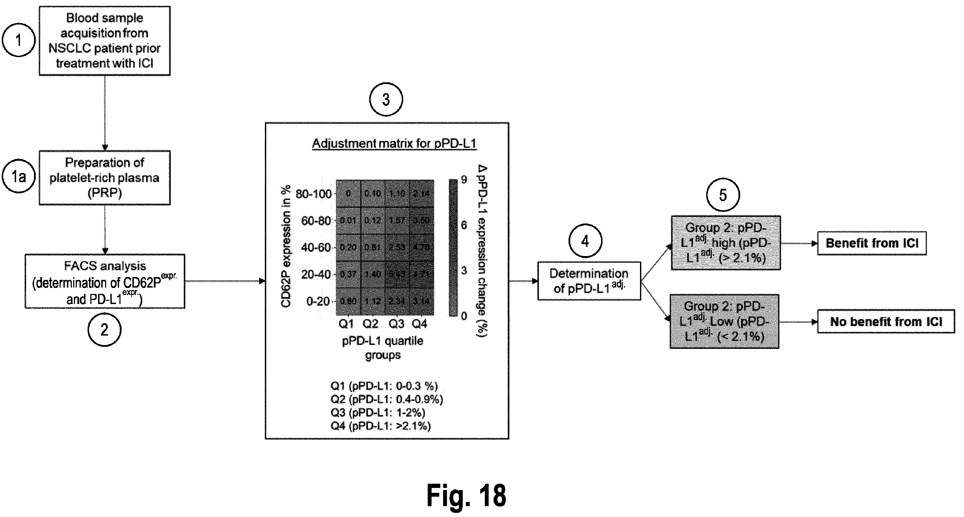

Fig. 18: Calculation and use of pPD-L1$^{adj.}$ and responsiveness prognosis in a clinical setting.

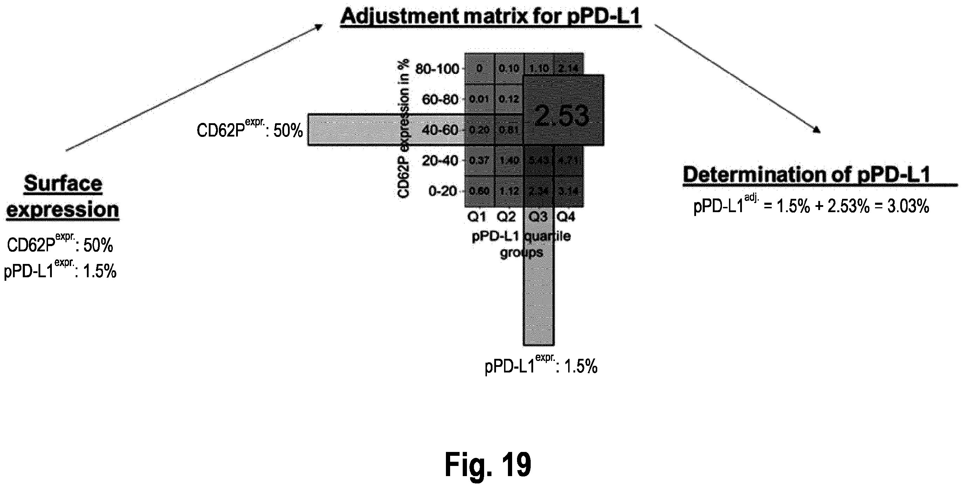

Fig. 19: Exemplary calculation of pPD-L1$^{adj.}$ and responsiveness prognosis.

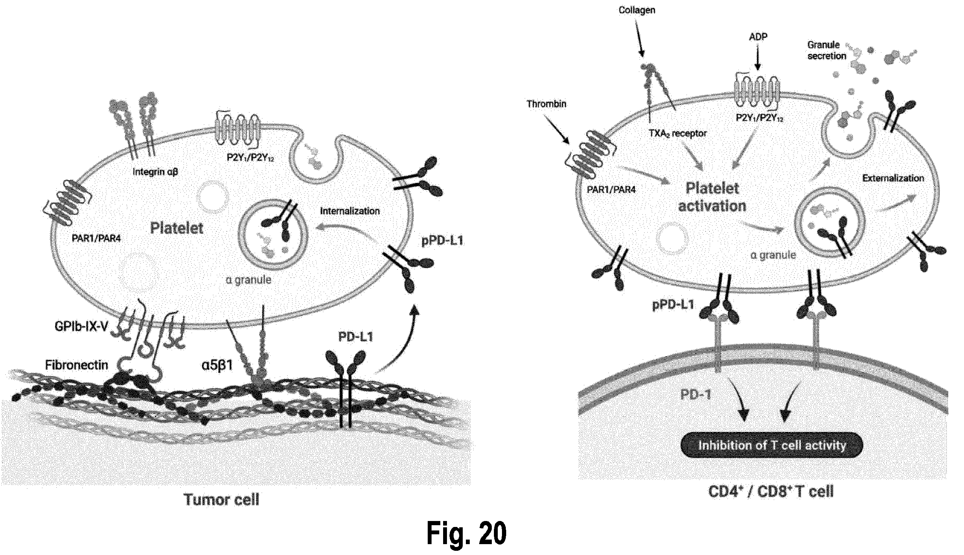

Fig. 20: Proposed model of pPD-L1 transfer from tumor cells to platelets and immunologic functions of pPD-L1. Tumor cells expressing fibronectin enhance tumor cell platelet crosstalk mediated via platelet GPIb-IX-V and integrin $\alpha5\beta1$ and lead to a consecutive uptake of PD-L1 from the tumor cell. In platelets, pPD-L1 is expressed on the platelet surface and stored intracellularly in $\alpha$-granules. Platelet activation via thrombin, ADP or collagen increase pPD-L1 expression on the platelet surface and finally suppress T cell reactivity. The graphic was created using BioRender (BioRender.com, Toronto, Canada).

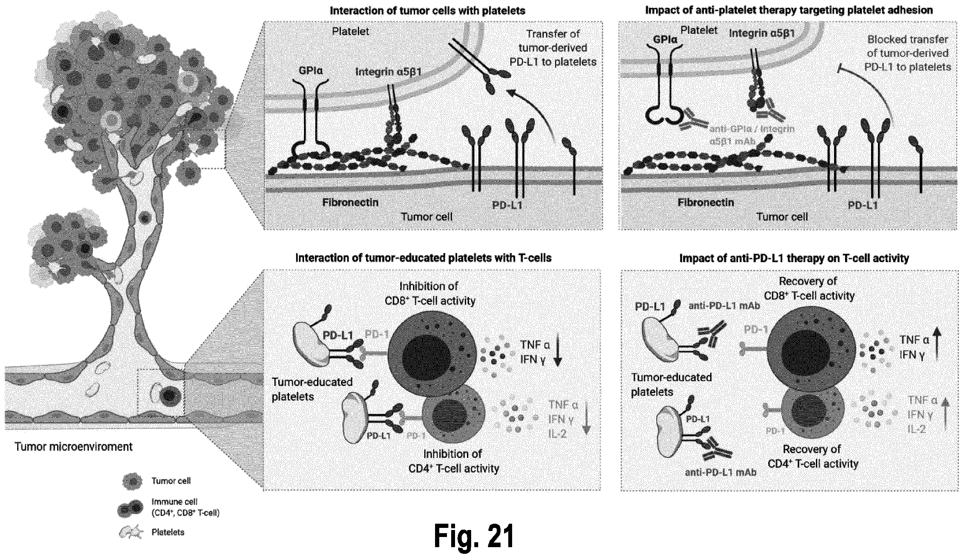

Fig. 21: Proposed model of the role pPD-L1 in the microenvironment and potential therapeutic interventions. In the TME platelets frequently interact with tumor cells and ingest tumor PD-L1 in a fibronectin, GPIb-IX-V and integrin $\alpha5\beta1$ dependent manner. Blocking of GPIb-IX-V and integrin $\alpha5\beta1$ reduce the uptake of PD-L1 and lower the immune inhibitory capacity of platelets in the TME. Blocking of pPD-L1 via anti-PD-L1 mAbs might contribute to the recovery of the anti-tumor T cell activity. The graphic was created with Bio-Render software (BioRender.com, Toronto, Canada).

EMBODIMENTS

1. Material and methods

*Study design and selection of patients*

During 2016-2019, 173 consecutive patients with non-small lung cancer (NSCLC) treated in the Department of Medical Oncology and Hematology and Department of Internal Medicine VIII, University Hospital Tuebingen, Germany were prospectively included in the study (screening cohort = SC). In order to preclude the influence of anticoagulants like aspirin (ASS), low molecular weight heparin (LMWH) or other heparinoids and non-vitamin K antagonist oral anticoagulants (NOACs), long-term medication of each patient was considered. In the inventors' cohort 12 patients with LMWH and 28 patients taking ASS and/or clopidogrel were excluded. In Fig. 16, a detailed flowchart of patient selection is given. In all cases sample collection was performed prior to the next application of the respective therapy. Tumor characteristics are based on baseline clinical staging. In order to take disease progression better into account the occurrence of metastasis was double checked at the time point of study inclusion. The inventors' cohort comprised 71 male and 57 female patients with a mean age of 65.7 years (range 19-87). The diagnosis of a NSCLC was histologically confirmed in all cases. NSCLC adenocarcinoma was identified in 93 patients (72.7%), in 35 cases (27.3%) a squamous cell carcinoma was found. The details of the all patients' characteristics are summarized in Table 1.

Table 1: Patients characteristics of the proof of principle (PoP) cohort

| Patient characteristics | | Total (n = 128) |
|---|---|---|
| **Gender** | | |
| | male sex, n (%) | 71 (55.5) |
| **Age** | | |
| Age at study inclusion in years, mean-yr. (95 % CI) | | 65.7 $\pm$ 14.3 (19 to 87) |
| **Histopathological subtype, n (%)** | | |
| | Adenocarcinoma | 91 (71.1) |
| | Squamous cell carcinoma | 35 (27.3) |
| | Large cell carcinoma | 2 (1.6) |
| | Unspecified | 0 |
| **TNM classification, n (%)** | | |
| | Stage | |
| | Tx | 15 (11.7) |
| | T1 | 11 (8.6) |
| | T2 | 30 (23.4) |
| | T3 | 24 (18.8) |
| | T4 | 48 (37.5) |
| | Node | |
| | Nx | 9 (7) |
| | N0 | 11 (8.6) |
| | N1 | 13 (10.2) |
| | N2 | 39 (21.7) |
| | N3 | 56 (43.8) |
| | Metastasis | |
| | M0 | 22 (17.2) |
| | M1 | 106 (82.8) |
| | **UICC stage, n (%)** | |
| | I | 1 (0.8) |
| | II | 6 (4.7) |
| | III | 13 (10.2) |
| | IV | 108 (84.4) |
| **Localization in lung on CT, n (%)** | | |

(continued)

| Patient characteristics | | Total (n = 128) |
|---|---|---|
| | Central | 43 (33.6) |
| | Peripheral | 71 (55.5) |
| | Not available | 14 (10.9) |
| **Smoking status, n (%)** | | |
| | Current or former smoker | 96 (75) |
| | Never smoked | 14 (10.9) |
| | Unknown | 18 (14.1) |
| **Genetic EGFR aberration, n (%)** | | 19 (14.8) |
| **Genetic ALK aberration, n (%)** | | 5 (3.9) |
| **Genetic ROS aberration, n (%)** | | 6 (4.7) |
| **Genetic KRAS aberration, n (%)** | | 38 (29.7) |
| **Number of prior systemic therapy, n (%)** | | |
| | 1 | 72 (56.3) |
| | 2 | 31 (24.2) |
| | ≥ 3 | 25 (19.5) |
| **Type of prior therapy, n (%)** | | |
| | Surgery | 23 (17.9) |
| | Radiation | 4 (1.8) |
| | Chemotherapy | 104 (47.3) |
| | Tyrosine kinase inhibitor | 30 (13.6) |
| Anti-PD-1/PD-L1 therapy | | 75 (34.1) |

n = number, yr. = year, % = percentage, T = tumor, N = lymph node, M = metastasis,
x = undefined, UICC = Union for International Cancer Control, EGRF = Epidermal Growth Factor Receptor

Written informed consent was given in all cases. This study was approved by IRB (ethics committee of the Faculty of Medicine of the Eberhard Karls University Tuebingen) and of the University Hospital Tuebingen and was conducted in accordance with the Declaration of Helsinki; reference number 456/BO2.

*Preparation of platelets*

Platelets were obtained from healthy donors (not taking any medication for at least 10 days) and NSCLC patients after informed writing consent. Citrated blood was briefly centrifuged for 20 min at 120 x g, the upper fraction was harvested as platelet-rich plasma (PRP). Platelets were washed twice with citrate wash buffer (128 mmol/L NaCl, 11 mmol/L glucose, 7.5 mmol/L $Na_2HPO_4$, 4.8 mmol/L sodium citrate, 4.3 mmol/L $NaH_2PO_4$, 2.4 citric acid, 0.35% bovine serum albumin, and 50 ng/mL prostaglandin E1 (PGE1)). To avoid the influence of PGE1 on platelet-tumor cell and platelet-immune cell interaction, the inventors did not use PGE1 in their co-incubation experiments. For platelet activation 10 μM of the Thrombin Receptor Activator Peptide 6 (TRAP-6), a protease-activated receptor 1 (PAR1) agonist, 2.5 μM ADP or 5 μg/mL Collagen was added to the platelets for 2 minutes. Platelets were fixed by 2% paraformaldehyde for 10 min and washed twice with PBS containing 1% FCS.

*Flow cytometry*

Flow cytometry was performed using fluorescence-conjugates or specific mAb and their controls followed by species-specific conjugate (Table 2) using a FACS Cantoll flow cytometer (Beckman Coulter) or a LSRFortessa (Becton Dickinson) from the flow cytometer facility Tuebingen.

**Abbreviations:**

BV: Brilliant violet
FITC: Fluorescein isothiocyanate

14

APC: Allophycocyanin
PE: Phycoerythrin
Cy: Cyanine
VB: Vioblue

Table 2: Antibodies

| Antibody | Clone | Fluorchrome | Vendor |
|---|---|---|---|
| Anti-CD41a | REA386 | VB | Miltenyi Biotec |
| Anti-CD41a | HIP8 | PeCy5 | BioLegend |
| Anti-CD62P | AK-4 | FITC | ThermoFisher |
| Anti-PD-L 1 | MIH-1 | APC | ThermoFisher |
| REA control | REA293 | VB | Miltenyi Biotec |
| Anti-Human IgG | - | APC | abcam |
| Anti-Human IgG | - | FITC | abcam |
| Anti-Human IgG | - | PeCy5 | BD |
| Anti-PD-L 1 | 28-8 | - | Abcam |
| Anti-CD61 | SJ-19-09 | - | ThermoFisher |
| Anti-CD3 | OKT3 | BV-510 | BioLegend |
| Anti-CD56 | HCD56 | BV605 | BioLegend |
| Anti-CD45RO | HI100 | BV785 | BioLegend |
| Anti-CD4 | RPA-T4 | APC-Cy7 | BioLegend |
| Anti-CD8 | B9.11 | PE-Cy7 | Beckman Coulter |
| Anti-CD27 | M-T271 | PE-CF594 | BD Bioscience |
| Anti-CD28 | CD28.2 | PE-Cy7 | BioLegend |
| Anti-CD62L | DREG-56 | FITC | BioLegend |
| Anti-GFP | EPR14104 | - | Abcam |
| Anti-IFNy | 4SB3 | PE | BioLegend |
| Anti-TNFa | Mab11 | Pacific blue | BioLegend |
| Anti-GFP | EPR14104 | - | Abcam |
| Anti-Fibronectin | P1H11 | - | Novus Biologicals |
| Anti-Mouse IgG | - | Alexa Fluor 488 | ThermoFisher |
| Anti-Mouse IgG | - | Alexa Fluor 594 | ThermoFischer |
| Anti-Rabbit IgG | - | Alexa Fluor 488 | ThermoFisher |
| Anti-Rabbit IgG | - | Alexa Fluor 594 | ThermoFisher |
| Anti-CD3 | UCHT-1 | PECy5 | BD |
| Anti-CD19 | HIB19 | APC/Fire750 | BioLegend |
| Anti-CD4 | RPA-T4 | Pacific blue | BioLegend |
| Anti-CD8 | RPA-T8 | BV605 | BioLegend |
| Anti-CD16 | CB16 | FITC | invitrogen |
| Anti-CD56 | HCD56 | PECy7 | BioLegend |
| Anti-CD14 | M5E2 | BV785 | BioLegend |
| Anti-HLA-DR | 1.245 | BV650 | BioLegend |
| Anti-PD-1 | EH12.2H7 | APC | BioLegend |
| Anti-PD-L1 | B7-H1 | APC | BioLegend |
| Anti-CD69 | FN50 | PE | BD |
| Anti-migG1 | MOPC21 | PE | BD |
| Anti-migG1 | MOPC21 | APC | BD |
| Anti-CD2 | REA1130 | FITC | Miltenyi Biotec |
| Anti-CD3 | REA1151 | FITC | Miltenyi Biotec |
| Anti-Cytokeratin | REA831 | FITC | Miltenyi Biotec |
| Anti-PD-1 | REA1165 | FITC | Miltenyi Biotec |

Positive cells in percentage (%) were calculated as follows: *Surface expression in percent obtained with the specific antibody - surface expression in percent obtained with isotype control.* Platelets were preselected by $CD41a^+$ and $CD62P^-$ (resting) or $CD62P^+$ (activated). Data analysis was performed using FlowJo software (v.10). In order to verify the reproducibility of the inventors' flow cytometry system, the inventors performed a Bland-Altman analysis (Fig. 11 f). For immunophenotyping of PBMC subsets of lung cancer patients and healthy control donors were identified by counterstaining with CD3-PECy5 (BD biosciences, San Diego, CA), CD19-APC/Fire750, CD4-Pacific Blue, CD8a-BV605, CD56-PECy7, CD14-BV785, HLA-DR-BV650 (Biolegend, San Diego, CA) and CD16-FITC (Invitrogen). PD-1 and PDL-1 expression as well as activation levels were analyzed using a PD-1-APC or PDL-1-APC and a CD69-PE antibody (BD biosciences), respectively. Isotype controls were obtained from BD biosciences. Dead cells were excluded from analysis with LIVE/DEAD™ Fixable Aqua (Thermo Fisher Scientific, Waltham, MA).

*Histopathology, immunohistochemistry and immunofluorescence staining of paraffin embedded tissue samples*

Tissue samples were fixed in 4% formalin and paraffin-embedded (FFPE) at the Department of Pathology (University Hospital Tuebingen). The sections were cut briefly in 3 $\mu$m sections and stained with Hematoxylin/Eosin and CD61 (clone: 2C9.G3) following standard protocols. For immunofluorescence microscopy, sections were deparaffinized and hydrated in a first step. The heat-induced antigen retrieval method was performed using sodium citrate buffer (pH 6.0) for 30 min. Antigen blocking was performed with Blocking solution (Zytomed) for 60 min. Primary antibodies that were included anti-CD41, mouse, 1:250 (clone: HIP8) and anti-PD-L1, rabbit, 1:200 (clone:28-8). Secondary antibodies include Alexa-Fluor 594 labelled anti-rabbit (1:1000, Invitrogen) and Fluor 488 labelled anti-mouse (1:1000, Invitrogen). DAPI (1:1000, BioLegend) was used for nuclear staining prior mounting the slides with H-1500 Vectashield Hardset. Microscopic analysis was done with an Olympus BX63 microscope and a DP80 camera (Olympus).

*Immunofluorescence staining of platelets and tumor cells*

For immunostaining tumor cells and/or platelets were fixed in 2% PFA in PBS (pH 7.4) for 10 min at -20°C. After three washing steps in PBS cells were incubated with a BSA blocking solution (2% BSA, 0,2% Triton X-100, 0,1% Tween) for 1 hour. Primary antibodies were anti-PD-L1, rabbit (1:250, clone: 28-8), anti-CD41, mouse (1:400, clone: HIP8), anti-CD61, rabbit (1:250, clone: SJ-19-09), anti-GFP, rabbit (1:200, clone: EPR14104), anti-fibronectin, mouse (1:200, clone: P1H11); as secondary antibodies Alexa-Fluor 488/594 labelled anti-rabbit (1:1000, Invitrogen) and Fluor 488/594 labelled anti-mouse (1:1000, Invitrogen) were used. Afterwards slides were mounted in fluorescent mounting medium containing DAPI (1:1000, BioLegend) counter-stain. For the plasma membrane staining CellMaskTM (ThermoFisher) and Dil (ThermoFisher) was used according to manufactures instructions. For nuclear staining NucBlueTM (ThermoFisher) was used. Image acquisition was performed using an Olympus BX63 microscope and a DP80 camera (Olympus). Quantification of platelets, fibronectin and tumor cells were performed via counting fluorescence positive signals using an ImageJ script (v.1.52).

*Cyclic immunofluorescence staining* of *NSCLC patient samples*

Paraffin-embedded patient samples were cut in 2-5 $\mu$m slices and collected on object slides. Subsequently, sections were subjected to deparaffinization and rehydration. Slides were treated with xylene for 10 mins, followed by rehydration using an ethanol dilution series of 100%, 95%, 70%, 50% for 5 mins each. One last change was performed using deionized water. Heat-induced antigen retrieval was performed using a Sodium-Citrate buffer (10 mM Sodium citrate, 0.05% Tween 20, pH 6.0) and boiling the samples for 20 mins. Samples were cooled down and stored in MACSima™ Running Buffer (Miltenyi Biotec, 130-121-565) until initial DAPI staining (Miltenyi Biotec, 130-111-570). The MACSimaTM device is an ultra-high content cyclic IF device which allows for fully automated IF imaging. Iteratively, the device performs fluorescent staining with multiple labelled antibodies, image acquisition, and bleaching per cycle. Images were generated according to the manufacturer's instructions and analyzed with the Qi Tissue Image Analysis Software. For quantification at least two ROI were selected based on manual prestaining of DAPI.

*Tracking platelet tumor cell interaction using live cell imaging*

For live cell imaging analysis A549 cells (cultured as stated above) were used. Tumor cells were co-incubated with platelets at a platelet-tumor cell ratio of 1:1000. Platelets were added to the tumor cells directly prior image acquisition. Platelet-tumor cell interaction was analyzed using confocal microscopy at frame intervals of 30 seconds for up to 40 min (Leica SP8 SMD; Leica HC PL APO CS 40x/0.85) using a fixed focus. Cell positions were assigned by their center-of-mass coordinates.

*Electron microscopy and immunoelectron microscopy*

For transmission electron microscopy, platelets from one representative pPD-L1 high expressing NSCLC patient were used. Platelets were centrifuged and the resulting pellets were fixed for 24 hours in Karnovsky's fixative. As previously described, Ultrathin sections were examined with a LIBRA 120 (Zeiss) operating at 120 kV. For immunoelectron microscopy, platelets were fixed and embedded in Lowicryl K4M (Polysciences). Samples were stained with anti-PD-L1 antibody (Abcam) and examined using a LIBRA 120 transmission electron microscope (Zeiss) at 120 kV.

*ELISA*

Protein levels of PD-L1 were measured using a human PD-L1 ELISA kit (abcam, clone: 28-8) according to the recommendations of the manufacturer. All concentrations are expressed as means $\pm$ SEM of triplicates.

*Western blot*

Whole-cell extracts were prepared using RIPA buffer and protein concentration was analyzed using the BioRad Dc assay. 25-50 $\mu$g of protein were transferred to 10-15% SDS-Page and blotted on a PVDF membrane (Millipore) with a wet blot system. The membrane was blocked for 1 h at room temperature with Roti-Block, followed by overnight incubation with the following antibodies: anti-PD-L1, rabbit (1:2000, clone: 28-8), anti-fibronectin, mouse (1:250, clone: P1H11), anti-Vinculin, mouse (1:10,000, clone: hVIN-1), anti-a tubulin (1:10,000, clone 11H10) and anti-$\beta$ Actin (1:10,000, clone AC-15). Blots were visualized using ECL reagents (GE Healthcare) or the Super Signal West Kit (Thermo Scientific) and the ChemiDocTM MP Imaging System.

*Real-time PCR*

To determine mRNA abundance in several tumor cell lines the inventors extracted mRNA in TriFast (Peqlab) according to the manufacturer's instructions. After DNAse digestion reverse transcription of total RNA was performed using random hexamers (Roche Diagnostics) and SuperScriptII reverse transcriptase (Invitrogen). Amplification of the respective genes by real-time polymerase chain reaction (RT-PCR) was performed in a total volume of 20 $\mu$l using 40 ng of cDNA, 500 nM forward and reverse primer and 2x GoTaq® qPCR Master Mix (Promega) according to the manufacturer's protocol. Cycling conditions were as follows: initial denaturation at 95°C for 2 min, followed by 40 cycles of 95°C for 15 sec, 55°C for 15 sec and 68°C for 20 sec. For amplification the following primers were used (5 ->3 orientation): Fibronectin (FN1), fw ACCGTGGGCAACTCTGTCAA (SEQ ID NO: 1), rev CCCACTCATCTCCAACGGCA (SEQ ID NO: 2); Tissue factor (F3), fw GGCACGGGTCTTCTCCTACC (SEQ ID NO: 3), rev TGTCCGAGGTTTGTCTCCAGG (SEQ ID NO: 4); Von Willebrand Factor (VWF), fw CCTGCACCGACATGGAGGAT (SEQ ID NO: 5), rev CGTAAGTGAAGCCCGACCGA (SEQ ID NO: 6); Fibrinogen A (FBG), fw TGAAACGACTGGAGGTGGACA (SEQ ID NO: 7), rev CACGAGCTAAAGCCCTACTGC (SEQ ID NO: 8); GAPDH (GAPDH), fw TCGACAGTCAGCCGCATCTT (SEQ ID NO: 9), rev GCCCAATACGACCAAATCCGT (SEQ ID NO: 10). Real-time PCR amplifications were performed on a CFX96 Real-Time System (Biorad) and all experiments were performed in duplicate. The housekeeping gene GAPDH was used to standardize the amount of sample RNA.

*In vitro platelet-tumor cell co-incubation and platelet adhesion*

Tumor cells were coated with platelets as described previously with slight modifications. Briefly summarized, PRP was obtained from fresh whole blood by centrifugation for 20 minutes at 120g. Platelets were washed twice with citrate wash buffer (128 mmol/L NaCl, 11 mmol/L glucose, 7.5 mmol/L Na$_2$HPO$_4$, 4.8 mmol/L sodium citrate, 4.3 mmol/L NaH$_2$PO$_4$, 2.4 citric acid, 0.35% bovine serum albumin). In some experiments platelets were pretreated with 5 $\mu$g/mL anti-CD42b (clone: AK2), 20 $\mu$g/mL anti-integrin $\beta$1 (clone: P4C10) and anti-Integrin $\alpha$5 (clone: JBS5) or corresponding control IgG1 (20 $\mu$g/mL) for 30 minutes at 37°C and 7% CO$_2$. Tumor cells were incubated in platelets at a platelet-tumor cell ratio of 1:1000 for 30 minutes at 37°C and 7% CO$_2$. For immunofluorescence microscopy and FACS analysis cells were fixed in 2% PFA in PBS (pH 7.4) for 10 min at -20°C prior staining.

*Preparation of fibronectin matrices and platelet blocking*

To prepare fibronectin matrices, plates were coated with a human plasma fibronectin purified protein (R&D, Minneapolis, MN, USA), (concentration 50 $\mu$g/cm$^2$) for 120 min. For blocking of GPIb-IX-V complex, $\alpha$5$\beta$1 or GPIIbIIIa, washed platelets (8x10$^7$/mL) were pretreated with 5 $\mu$g/mL anti-CD42b (clone: AK2), 20 $\mu$g/mL anti-integrin $\beta$1 (clone: P4C10) and anti-Integrin $\alpha$5 (clone: JBS5), 1 $\mu$g/mL Tirofiban or corresponding control IgG1 (20 $\mu$g/mL) for 30 minutes at 37°C and 7% $CO_2$. After co-incubation with platelets ($8x10^7$/mL) for 30 min at 37°C and 7% $CO_2$, non-adherent platelets were removed via three washing steps using PBS. After removal of non-adherent platelets cells were fixed in 2% PFA in PBS (pH 7.4) for 10 min at -20°C. Platelet adhesion to fibronectin fibrils was evaluated by calculating surface coverage area and platelet count / FoV and from microscopic images using an ImageJ script (v.1.52).

*Plasmid construction and transfection of NSCLC cells*

For overexpression of PD-L1 (CD274) a True-ORF-GFP-tagged expression vector was used (OriGene Technologies, Rockville, MD, USA). Control cells were transfected using a FLAG tag. The FLAG cDNA was generated by PCR and cloned into the PD-L1-GFP vector using AsiSI and MluI restriction sites. Tumor cells were transfected with 2.5 $\mu$g DNA (PD-L1-GFP, FLAG-GFP) using LipofectamineTM 3000, in accordance to the manufacturer's instructions.

*Generation of peripheral blood mononuclear cells (PBMC) and tumor cell lines*

Peripheral blood mononuclear cells (PBMC) from healthy donors were isolated using Ficol/Paque (Biochrom) density gradient centrifugation after informed consent. All tumor cell lines were cultured with 10% FCS in Roswell Park Memorial Institute (RPMI) 1640 Medium at 37°C and 7% $CO_2$. Cell proliferation was quantified using a Neubauer chamber; for viability testing Trypan blue staining's was performed using a 0.4% trypan blue solution (Fluka). The tumor cell lines A549, NCI-H460, NCI-H23, NCI-H226, NCI-H322, NCI-H522, HOP-62 and HOP-92 were obtained from the American Type Culture Collection (ATCC). Mycoplasma contamination was excluded via a PCR-based method.

*IFN$\gamma$ ELISPOT assay in CD8$^+$ T cells and platelet-T-cell co-incubation*

Freshly thawed (*ex vivo*) PBMCs from healthy donors were analyzed by enzyme-linked immunospot (ELISPOT) assay in duplicates. Interferon $\gamma$ (IFN$\gamma$) ELISPOT assays in the inventors' study were performed as described previously. In brief, 96-well nitrocellulose plates (Millipore) were coated with 1 mg/mL anti-IFNy mAb (Mabtech) and incubated overnight at 4°C. In a next step, plates were blocked with human serum (10%) for 2 hours at 37°C. PBMCs ($2.5 \times 10^5$ cells per well) were pulsed with an EBV/CMV epitope mix containing the frequently recognized peptides BRLF109-117 YVLDHLIVV (SEQ ID NO: 11) (A*02) peptide and CMV pp65 (A*02) peptide NLVPMVATV (SEQ ID NO: 12) and incubated with or without platelets (ratio 1:50) for 24 hours. Phytohemagglutinin was used as positive control. HLA-A*02 (KLFEKVKEV (SEQ ID NO: 13))- and B*07 (KPSEKIQVL (SEQ ID NO: 14))-restricted control peptides derived from benign tissues (HV-exclusive HLA ligands) served as negative control. Prior co-incubation with T cells PD-L1 positive platelets from NSCLC patients were pre-treated with the anti-PD-L1 mAB Atezolizumab for 30 min and washed twice with PBS containing 1% FCS. Readout was performed according to the manufacturer's instructions. Spots were counted using an ImmunoSpot S5 analyzer (CTL).

*Cytokine and cell surface marker staining*

Peptide-specific T cells were further analyzed by intracellular cytokine and cell surface marker staining. PBMCs were incubated with 10 $\mu$g ml$^{-1}$ of peptide, 10 $\mu$g ml$^{-1}$ brefeldin A (Sigma-Aldrich) and a 1:500 dilution of GolgiStop (BD) for 12-16 h. Staining included Cytofix/Cytoperm solution (BD), anti-CD4, mouse (1:100, clone: RPA-T4), anti-CD8, mouse (1:400, clone: B9.11), anti-TNF, mouse (1:120, clone: Mab11) and anti-IFN-y, mouse (1:200 dilution, clone: 4SB3). PMA (5 $\mu$g ml$^{-1}$) and ionomycin (1 $\mu$M, Sigma-Aldrich) served as positive control. Viable cells were determined using Aqua live/dead (1:400 dilution, Invitrogen). Samples were analyzed on a FACS Canto II cytometer (BD) and evaluated using FlowJo software v. 10.0.8 (BD).

*Generation of NY-ESO-1-specific CD4$^+$ T cells and platelet-T-cell co-incubation*

The generation of NY-ESO-1-specific T cells was performed using as described previously. In brief, PBMCs from a healthy donor ($1x10^7$/mL) were stimulated using pools of NY-ESO-1 overlapping peptides (1 $\mu$g/mL). The NY-ESO-1 overlapping peptide pool of 15 amino acid length (11 amino acid overlap) was purchased via Miltenyi Biotec. The cells were cultured in RPMI 1640 containing 10 % human AB-serum and 1% L-glutamin in the presence of 10 U/mL recombinant IL-2 and 10 ng/mL Il-7. Culture medium was replaced every third day. After a pre-sensitization period of 7-14 days, NY-ESO-1 specific, IFN$\gamma^+$ T cells were enriched after re-stimulation with NY-ESO-1 peptide pool for 6 h using CliniMACS® (Miltenyi Biotec) technique as reported previously. After enrichment, NY-ESO-1 specific T cells were expanded for 14 days in the presence of IL-7 (10 ng/mL), IL-15 (10 ng/mL) and IL-2 (50 U/mL). T cell specificity was analyzed via intracellular IFN$\gamma$ staining as stated above. For further characterization of the T-cells the differentiation markers anti-CD45RO, mouse (1: 200, clone: HI100), anti-CD62L, mouse (1:400, clone: DREG-56), anti-CD28, mouse (1:200, clone: CD28.2) and anti-CD27, mouse (1:200, clone: M-T271) were co-analyzed by flow cytometry. For the platelet-T-cell co-incubation assay, NY-ESO-1 specific T cells ($5 \times 10^6$/mL) were cultured in TexMACS GMP Medium (Miltenyi Biotec). Six hours prior analysis T cells were co-incubated with platelets of NSCLC patients or healthy donors (ratio 1:200) and re-stimulated with NY-ESO-1 peptides (1 $\mu$g/mL). In order to investigate the functional role of PD-L1 on platelets surfaces, PD-L1 positive platelets from NSCLC patients were pre-treated with Atezolizumab (100$\mu$g/mL) for 30 min and washed twice with PBS containing 1% FCS. As a negative control a Myelin oligodendrocyte glycoprotein (MOG) peptide mix was used (1 $\mu$g/mL). SEB (Toxin Technology, Sarasota, FL, USA) at 10 $\mu$g/mL was used as positive control. NY-ESO-1 specific T cell activity was determined by intracellular TNF$\alpha$ and IFN$\gamma$ quantified via flow cytometry as described above.

*In situ proximity ligation assay (PLA)*

HOP-62 and NCI-H23 cells were grown on glass bottomed plates. After two washing steps cells were fixed in 1% PFA in PBS (pH 7.4) for 10 min at -20°C. After three washing steps in PBS cells were incubated with a BSA blocking solution (5% BSA, 0,2% Triton X-100, 0,1% Tween) for 30 min. In situ PLA was performed using the Duolink PLA kit (Sigma-Aldrich) according to the manufacturer's instructions. In brief, after blocking cells were incubated with anti-PD-L1, rabbit (1:250, clone: 28-8) and anti-fibronectin, mouse (1:200, clone: P1H11) for 2 h at room temperature. After three washing steps with PBST (phosphate buffered saline, 0.1% Tween), anti-mouse PLUS and anti-rabbit MINUS PLA probes were linked to the primary antibodies for 1 h at 37°C. After three times washing steps with buffer A (0.01 M Tris, 0.15 M NaCl, and 0.05% Tween-20), PLA probes were ligated for 60 min at 37°C. After two washing steps with buffer A, amplification using Duolink In Situ Detection Reagents (Sigma) was performed at 37 °C for 120 min. Following amplification, cells were washed three times for 5 min with wash buffer B (0.2 M Tris 0.1 M NaCl). Cells were than coated with Duolink Mounting Medium containing DAPI. Image acquisition was performed using an Olympus BX63 microscope and a DP80 camera (Olympus).

*Establishment of an activation-independent calculation matrix for platelet PD-L1*

Since platelet pre-activation levels differ due to sample collection/preparation and protein surface expression depends on the platelet activation state, accurate determination of total protein expression on platelet surfaces is challenging. As a result, the platelet pre-/activation level acts as a confounding factor and thus impairs the suitability of pPD-L1 as a promising biomarker in NSCLC. To circumvent this dilemma, the inventors established an activation-independent calculation matrix of platelet PD-L1. The matrix is based on the inventors' cohort of 128 NSCLC patients and investigates the activation-dependent expression change of PD-L1 ($\Delta$pPD-L1) during controlled platelets stimulation *ex vivo*. Patients were categorized into pPD-L1 quartile groups (Q1: very low, Q2: low, Q3: medium, Q4: high), according to the pPD-L1 expression in unstimulated platelets. The pre-activation of platelets after sample preparation was determined via CD26P expression. In a second step each quartile group was subdivided according to the respective pre-activation levels (CD62P expression: 0-20%, 20-40%, 40-60%, 60-80%, and 80-100%) according to the pre-activation levels. The activation-dependent expression changes of PD-L1 ($\Delta$pPD-L1) was then calculated for each subgroup. An overview of the subsampling and calculation is given in Fig. 19.

*Statistics*

Student's t test, Mann-Whitney U test, one-way ANOVA and Friedman's test were used for continuous variables, chi-squared test or Fisher's exact test for categorical variables. If significant differences by ANOVA were found, group wise comparison was done (Tukey's multiple comparison test). If significant differences by Friedman's test were found Dunn's multiple comparisons test was used. Overall survival (OS) and progression free survival (PFS), including the median, were calculated using the Kaplan-Meier method. Hazard ratios (HRs) were determined using Cox regression analysis. OS was calculated from the date of primary diagnosis or time-point of study inclusion and stratified by the end of the study. The predictive value of platelet-derived PD-L1 as a prognostic factor was evaluated by examining the area under the receiver-operator characteristic (ROC) curve using a confidence interval of 95%. All statistical tests were considered statistically significant when P was below 0.05. Statistical analysis was performed using SigmaStat, version 21 (SPSS) and GraphPadPrism (v.8.1.0).

## 2. Results

*Tumor cells transfer PD-L1 to platelets*

To address whether the immune regulatory protein PD-L1 can be transferred from tumor cells to platelets, the inventors co-incubated platelets obtained from healthy donors with four different NSCLC tumor cell lines harboring varying expression levels of PD-L1 (NCI-H23, A549: PD-L1 low/negative, NCI-H226, NCI-H460: PD-L1 positive) (Fig.1 a, b). PD-L1 positivity was determined by flow cytometry and defined as PD-L1 expression in $\geq$ 5% of all tumor cells. PD-L1 expression on platelets (pPD-L1) was observed after co-incubation with the PD-L1 expressing NCI-H226 and NCI-H460 cells but not after co-incubation with the PD-L1 low/negative cell lines NCI-H23 and A549 (Fig. 1 c-e). Results were validated using a flow cytometry-based approach (Fig.1 f). Co-incubation of platelets with all tumor cell lines resulted in platelet activation, as indicated by P-selectin (CD62P) induction (Fig. 1 g), however only co-incubation with PD-L1 positive NCI-H226 and NCI-H460 cells resulted in an increased PD-L1 expression on the platelet surface (Fig.1 h). To ensure that platelets from healthy donors, used in this assay, do not harbor relevant amounts of endogenous PD-L1 the inventors conducted western blot analyses on platelet whole cell lysates. Indeed, Western Blot data confirmed that platelets from healthy donors do not express relevant PD-L1 levels (Fig. 2 b).

Of note, conditioned medium from tumor cells induced platelet activation but did not result in increased levels of PD-L1 protein on the platelet surface (Fig.3 c-d), suggesting that PD-L1 transfer from tumor cells to platelets is dependent on a direct cell-cell contact between both cell types. Of note, frequent interaction with platelets was not restricted to adherent tumor cells but could for example also be observed for non-adherent A549 lung cancer cells (Fig. 3 e-f).

To gain deeper insights into the interaction of platelets and lung cancer cells, the inventors took advantage of a live cell imaging platform, where platelets are added to the medium and circulate through an imaging chamber that contains human NSCLC cells. Real time video microscopy revealed distinct interactions of tumor cells and platelets (Fig. 1 i, j). Strikingly, platelets remained fully agile and re-entered the circulation after contacting the tumor cell membrane (Fig. 1 k-j). These data suggest that platelets can re-circulate after tumor cell attachment and activation and are in line with studies by Cloutier and Michaelson et al.

While platelets are anuclear, protein translation from RNA can nevertheless occur within platelets. The inventors therefore set out to investigate whether PD-L1 expression in platelets depends on a transfer of PD-L1 protein from tumor cells to platelets or whether a transfer of PD-L1 mRNA with subsequent protein synthesis within the platelet is involved. Transfection of vectors encoding for PD-L1-GFP and FLAG-GFP fusion proteins into PD-L1 negative A549 cells (Fig.1 m-o) resulted in high numbers of GFP positive platelets upon co-incubation (Fig.1 p-s). As inhibition of protein translation in platelets by cycloheximide did not result in a reduction of PD-L1-GFP expression in platelets (Fig. 1 t), the inventors' data suggest that PD-L1 protein transfer and not mRNA transfer is underlying the observed pPD-L1 expression after interaction of platelets and tumor cells.

While the transfer of PD-L1-GFP or FLAG-GFP was robustly observed across various NSCLC cell lines, the inventors nevertheless noted differences in protein transfer efficacies. For example, platelets showed low levels of FLAG-GFP and PD-L1-GFP after co-incubation with NCI-H322, NCI-H522 and NCI-H23 cells, while HOP-62 and HOP-92 cells displayed significantly higher protein transfer rates (Fig. 4 a-d). Given that the inventors' data indicated that a platelet-tumor-cell contact is necessary for a sufficient transfer of PD-L1 from tumor cells to platelets, the inventors hypothesized that expression levels of adhesion molecules might determine the efficacy of protein transfer from tumor cells to platelets. Along these lines the inventors found that PD-L1 transfer rates positively correlated with fibronectin (FN) mRNA expression levels, while no significant correlation was found for fibrinogen alpha chain (FGA) or tissue factor (F3) mRNA expression (Fig. 4 e-g). Of note, fibronectin expression also correlated with platelet tumor cell interaction *in vitro* (Fig. 4 h-j). Immunofluorescence staining as well as analysis of protein-protein interaction via proximity ligation assay (PLA) revealed close proximity of fibronectin and PD-L1 (Fig. 4 k-n) at the cell surface. In line with these observations, the inventors found that siRNA mediated knockdown of fibronectin resulted in a significant reduction of PD-L1 transfer, thus functionally validating fibronectin as a key regulator of protein transfer from tumor cells to platelets (Fig. 4 o-q). Platelet adhesion to fibronectin is known to be mediated via several molecules including GPIb$\alpha$, integrin $\alpha5\beta1$ or GPIIbIIIa. The inventors therefore set out to address whether inhibition of these adhesion molecules on platelets reduces adhesion to fibronectin and PD-L1 uptake from tumor cells. Strikingly, while monoclonal antibodies against GPIb$\alpha$ and integrin $\alpha5\beta1$ prevented platelet adhesion to fibronectin (Fig. 4 r-t) and PD-L1 protein transfer (Fig. 4 u-v), inhibition of GPIIbIIIa by Tirofiban only marginally reduced platelet adhesion (Fig. 4 r-t).

*Detection of functional PD-L1 on platelets of NSCLC patients*

To address the significance of the inventors' findings for human cancers, the inventors next quantified PD-L1 expression on platelets in healthy lung tissue or NSCLC tumor tissue. While platelets were detected in high abundance in healthy lung tissue and PD-L1 negative NSCLC, the inventors could not observe any relevant PD-L1 expression on these platelets (Fig. 5 a-b, d-e). In contrast PD-L1 positive platelets were observed in high abundance in tissue sections from patients suffering from PD-L1 positive NSCLC (Fig. 5 c, d-e). To quantify the number of PD-L1 positive platelets outside the tumor, the inventors next isolated platelets from the peripheral blood of a cohort of 64 healthy donors and 128 NSCLC patients. Fluorescence-Activated Cell Sorting (FACS) revealed threefold higher numbers of PD-L1 positive platelets in NSCLC patients as compared to healthy donors (median pPD-L1 expression in healthy donors 0.29 (95%CI: 0.21 - 0.44), median pPD-L1 expression in NSCLC patients 0.89 (95%CI: 0.61 - 1.21), (Fig. 5 f). The detected differences were even higher, when total pPD-L1 levels were determined using a quantitative enzyme-linked immunosorbent assay (ELISA). While platelet rich plasma (PRP) from NSCLC patients in average contained 108.3 pg/mL PD-L1, PRP from healthy volunteers only contained 1.8 pg/mL (Fig. 5 g-h). Differences in pPD-L1 expression in NSCLC patients versus healthy volunteers was also confirmed using western blot analysis (Fig. 2 b-d). Interestingly, PD-L1 expression was highest in patients with advanced (UICC stage IV) tumors (Fig. 2 e). Of note, immunofluorescence (Fig. 5 i) and immunoelectron microscopy (Fig. 5 j) revealed frequent PD-L1 clusters in platelets obtained from peripheral blood of a PD-L1 positive NSCLC patient, further underlining functionality of pPD-L1, as immune ligand clustering has been described to be a prerequisite for proper binding to its receptor.

Prompted by these results, the inventors next explored whether pPD-L1 exerts immune-inhibitory functions. The inventors stimulated human T cells from healthy donors with EBV/CMV-derived peptides in the presence or absence of PD-L1 positive platelets obtained from NSCLC patients. T cell activation was evaluated using an enzyme-linked-immuno-Spot (ELISpot) assays determining the effector cytokines IFN$\gamma$ and TNF$\alpha$. In line with published data the inventors observed that platelets dampen T cell activity independent of their PD-L1 expression status (Fig. 6 a-c and Fig. 7 a, b). However, when PD-L1 expressing platelets were pre-treated with the anti-PD-L1 mAb Atezolizumab their T cell inhibitory effect was abolished (Fig. 6 a-c). Next, the inventors expanded their work towards tumor associated antigens. New York esophageal squamous cell carcinoma 1 antigen (NY-ESO-1) belongs to the family of cancer-testis antigens, but is also aberrantly expressed in many tumor entities including NSCLC. Stimulation of T cells from healthy donors with NY-ESO-1 peptides predominantly resulted in a clonal expansion of NY-ESO-1 specific CD4$^+$ T cells (CD62L$^-$/CD45RO$^+$ and CD27$^-$/CD28$^+$) (Fig. 6 d), which were further specified as CD4$^+$ effector memory T cells (TEM, CD62L$^-$/CD45RO$^+$ and CD27$^-$/CD28$^+$) (Fig. 6 d-e). Remarkably, TEM activity, as determined by IFN$\gamma$ and TNF$\alpha$ release, decreased significantly upon co-incubation with PD-L1 positive platelets. However, T cell activity could be restored when pPD-L1 positive platelets were pretreated with anti-PD-L1 (Fig. 6 f-k).

To investigate a potential impact of PD-L1 positive platelets on other immune cells, the inventors also characterized changes in the overall immune cell composition (peripheral blood) in 10 NSCLC patients and five healthy controls (Fig. 8 a). In NSCLC patients pPD-L1 tended to be correlated negatively with the total number of NK (p=0.1), CD4$^+$ T cells (p=0.09) and CD8$^+$ T cells (p=0.02) (Fig. 8 b). Moreover, in NSCLC patients more PD-1 and PD-L1 was expressed on dendritic cells (DCs), natural killer (NK) cells and CD4$^+$ and CD8$^+$ T cells (Fig. 8 c-d). In the inventors' analyses they didn't observe a correlation of PD-1 or PD-L1 expression and pPD-L1 in DCs, NK cells or CD4+ T cells (Fig. 8 e-f). However, we detected a positive correlation of pPD-L1 and PD-1 on CD8$^+$ T cells (p=0.02). The inventors also quantified T cells and T cell infiltration in the TME in eleven NSCLC patients with different levels of pPD-L1 using the MACSima ultradeep tissue profiling platform. Noteworthy, in patients with high pPD-L1 the inventors observed lower numbers of T cells in the TME and less infiltrating T cells (Fig. 9 a-d). In contrast to their findings in the peripheral blood, the inventors observed an inverse correlation of PD-1 on T cells and pPD-L1 (Fig. 9 e-f).

*Regulation of pPD-L1 during platelet activation*

As it is well established that expression levels of platelet surface proteins correlate with the platelet activation status, the inventors reasoned that different degrees of platelet activation might underlie varying levels of pPD-L1 expression on the platelet surface. Indeed, when the inventors analyzed the platelet activation marker CD62P, the inventors observed varying CD62P expression levels which showed a strong positive correlation with pPD-L1 expression (Fig. 10 a). Of note, while PD-L1 expression in general was lower in unstimulated platelets, the inventors were able to robustly detect pPD-L1 on the platelet surface of resting (CD62P negative) platelets (Fig. 2 a). In line with this, the inventors also detected pPD-L1 in $\alpha$-granules (Fig. 10 b).

As even highly standardized blood collection procedures can result in varying levels of shear-stress mediated platelet activation and therefore complicates standardization, the inventors hypothesized that different levels of platelet pre-activation might complicate the interpretability and comparability of pPD-L1 levels on freshly collected platelets from different patients. The inventors therefore reasoned that a controlled *in vitro* activation of platelets with subsequent maximization of pPD-L1 expression might most adequately uncover the total payload of platelet PD-L1 and best possibly allow a comparison between different patients. Indeed, the inventors found that pPD-L1 expression was maximized upon controlled platelet stimulation with the PAR1 agonist TRAP-6 (Fig. 10 c-f and Fig. 11 a-d) or other platelet activation agents such as ADP or collagen (Fig. 10 g-h) and thus might allow for a better comparability of pPD-L1 levels between different patients. However, controlled platelet activation and subsequent measurement of CD62P and pPD-L1 is technically demanding and might prevent the use of pPD-L1 as a biomarker in clinical routine. The inventors therefore set out to explore whether a "normalized" PD-L1 level on the platelet surface could be calculated without *in vitro* manipulation of platelets. To do so the inventors developed an adjustment model based on the calculation of $\Delta$PD-L1 (ratio of PD-L1 before and after stimulation) as a function of pPD-L1 expression in unstimulated platelets and the degree of pre-activation (CD62P expression) (Fig. 10 i-j). Specifically, the inventors devised a matrix which allows to calculate PD-L1$^{adj.}$ for subgroups of patients harboring different levels of platelet pre-activation (CD62P expression) and pPD-L1 expression (Fig. 10 j and Fig. 12). Taking advantage of the inventors' matrix, corrected PD-L1 levels, designated pPD-L1$^{adj.}$, were determined for all patients (Fig. 10 k).

*Adjusted platelet-derived PD-L1 serves as a prognostic and predictive marker in NSCLC*

The inventors first used the calculated pPD-L1$^{adj.}$ levels and performed a receiver operating characteristic (ROC) analysis for overall survival (OS). The inventors found that pPD-L1$^{adj.}$ levels in the subgroup of maximal platelet activation (CD62P 80-100%) showed highest accuracy in predicting OS (Fig. 13 a). Since no cut-off value for pPD-L1$^{adj.}$ had been established so far, the inventors analyzed OS in pPD-L1$^{adj.}$ quartile groups (Q1-4) using the Kaplan-Meier method (Fig. 13 b, c). Details regarding characteristics of the inventors' patient population are provided in Table 1. The median observation time for monitoring OS in the inventors' study was 23.5 months (95%CI: 3.4-67.55 months). At data cutoff for overall survival, 42 of 128 patients (32.8%) were still alive. Strikingly, patients with high pPD-L1$^{adj.}$ levels showed a significantly shortened OS (Fig. 13 b). The median survival in Q1 (low pPD-L1$^{adj.}$ levels) was 43 months compared to only 24 months in Q3 (high pPD-L1$^{adj.}$ levels) (hazard ratio (HR) for death Q1 vs. Q3: 2 (95%CI: 1-3.9)) and 14 months in Q4 (very high pPD-L1$^{adj.}$ levels) (hazard ratio (HR) for death Q1 vs. Q4: 3.64 (95%CI: 1.97-6.72)). Importantly, the observed differences in OS were not restricted to the time since initial diagnosis but were still significant when analyzing the time period since platelet analysis (Fig. 13 c).

It has been reported that mutations in key oncogenic drivers do not only fuel proliferation via cell intrinsic cues but also impact tumor biology via modulation of the tumor microenvironment. Along these lines, the inventors found increased pPD-L1$^{adj.}$ levels in patients suffering from KRAS mutated NSCLC as compared to those with KRAS wildtype status (Fig.13 d). In contrast, mutations in EGFR, ALK fusions and ROS1 fusions or mutations showed no association with pPD-L1$^{adj.}$ levels, respectively (Fig. 13 d-e).

The inventors also explored a potential correlation of pPD-L1adj. with other clinical parameters. For example, the inventors found that patients with higher tumor stages (T, p=0.03), higher degrees of lymph node invasion (N, p=0.04) and a higher tumor grading (G, p=0.002) expressed more PD-L1 on the platelet surface (Fig. 13 f-h). No association was found between pPD-L1$^{adj.}$ and the region of tumor origin (central vs. peripheral, Fig. 13 i). However, pPD-L1$^{adj.}$ strongly correlated with the occurrence of metastases (p<0.001), especially liver (p=0.005) and brain metastasis (p=0.001) (Fig. 13 j-l). In line with previous studies, the inventors also found pPD-L1$^{adj.}$ to be positively correlated with smoking history and the amount of pack years (Fig. 14 c-d). Moreover pPD-L1$^{adj.}$ was positively correlated with platelet count, LDH and CRP (Fig. 14 j-l).

To further elaborate on the potential of pPD-L1Adj. as a predictive biomarker in NSCLC, the inventors conducted sequential measurements of pPD-L1$^{adj.}$ in 12 patients undergoing conventional chemotherapy or ICI (Fig. 15 a-b). Details on therapeutic regimens are provided in Fig. 16. In these patients baseline pPD-L1$^{adj.}$ levels were determined prior to the first cycle of the respective 1st line treatment. The second measurement was conducted in parallel to the first CT scan. Remarkably, a significant drop in pPD-L1$^{adj.}$ levels was detected upon initiation of therapy in those patients whose tumors were later identified to have undergone at least partial remission (PR), (p=0.02, Fig. 15 a). In contrast, patients who were later identified to have progressed despite therapy displayed a significant increase of pPD-L1$^{adj.}$ already in early measurements after therapy initiation (p=0.04, Fig. 15 b). Of note, the predictive value of pPD-L1$^{adj.}$ was robust regardless of the used therapeutic regime. In two patients receiving ICI the inventors determined pPD-L1$^{adj.}$ at multiple time points. Remarkably, pPD-L1$^{adj.}$ expression changes correlated with disease activity routinely determined via CT-scan (Fig. 15, c-f). As genomic alterations in EGFR and ALK represent independent factors influencing OS and progression-free survival (PFS), especially in patients receiving ICI, the inventors additionally analyzed the role of pPD-L1$^{adj.}$ in the respective subgroups with or without such alterations. Whereas the inventors were not able to detect a significant difference regarding OS (Fig. 17 a-c), in pPD-L1$^{adj.}$ high patients harboring an EGFR or ALK alteration who received a platinum-based chemotherapy, PFS tended to be worse compared to patients without EGFR and ALK alteration (Fig. 17 d-e). This might be explained by the fact that these patients had already shown a tumor progression upon first-line treatment with a tyrosine kinase inhibitor (TKI). In patients with EGFR and ALK alteration who received TKI, pPD-L1$^{adj.}$ was not predictive for PFS (Fig. 17 f). Since in the inventors' cohort none of the patients receiving ICI showed EGFR or ALK aberrations, the role of pPD-L1$^{adj.}$ could not be investigated in this cohort.

Subsequently, the inventors set out to probe whether the pre-therapeutically determined pPD-L1$^{adj.}$ level can predict the therapy response of NSCLC patients to immune checkpoint blocking antibodies. To do so the inventors analyzed the PFS of patients either treated with only conventional chemotherapy or immunocheckpoint blockade. pPD-L1 positive and negative subgroups were defined according to the median pPD-L1adj. level. In patients receiving conventional chemotherapy the inventors observed a significantly higher PFS when pPD-L1 levels were low (Fig. 13 m). Interestingly, in patients treated with ICI (Pembrolizumab or Nivolumab), high pPD-L1$^{adj.}$ predicted a PFS benefit (HR 4.74, p=0.003) (Fig. 13 n and Fig. 17 j). Strikingly, when the predictive power of pPD-L1$^{adj.}$ was compared to conventional histological PD-L1 quantification (TPS >50% and $\geq$1% in tumor biopsies), pPD-L1$^{adj.}$ was found to much better predict therapy response towards ICI (Fig. 13 o, Fig. 17 k-l). In summary, the inventors' data suggest that pre-therapeutically measured pPD-L1$^{adj.}$ levels accurately predict the therapeutic response towards immune checkpoint blocking antibodies. Prospective clinical trials are warranted to validate the inventors' findings and to justify the implementation of pPD-L1$^{adj.}$ as a biomarker in clinical routine.

*Calculation and use of pPD-L1$^{adj.}$ in a clinical setting for predicting a patient's benefit from therapy with an immune checkpoint inhibitor*

In Fig. 18 a scheme for the calculation of pPD-L1$^{adj.}$ and the carrying-out of the method according to the invention in daily clinical routine is illustrated. In step 1 a platelets-containing sample such as a blood sample is acquired from a patient, e.g. an NSCLC patient, prior treatment with ICI. In step 1a of the method according to the invention platelet-rich plasma (PRP) is prepared as known by the skilled practitioner or as described further above. In step 2 the platelets are analyzed via flow cytometry or fluorescence activated cell sorting (FACS) for the expression of a platelet activation marker, such as CD62P, and for the expression of PD-L1 (CD274), to obtain CD62P$^{expr.}$ and pPD-L1$^{expr.}$, respectively. In step 3 the adjustment procedure for pPD-L1 is carried out to obtain pPD-Li$^{corr.}$. From the matrix, based on the expression levels determined for the platelet activation marker (CD62P$^{expr.}$) and for pPD-L1 (pPD-L1$^{expr.}$), the corresponding correction value pPD-L1$^{corr.}$ is selected. For this purpose, one selects in the y-direction the column corresponding to the range of CD62P expression in which the measured CD62P$^{expr.}$ falls. Furthermore, one selects in x-direction the quartile corresponding to the range of pPD-L1 expression in which the measured pPD-L1$^{expr.}$ falls. The box located at the intersection of both selection processes contains the searched value pPD-L1$^{corr.}$. In step 4 pPD-L1$^{adj.}$ is determined by adding pPD-L1$^{expr.}$ and pPD-L1$^{corr.}$. In step 5 the patient is classified into high vs. low group associated with a prognosis of the responsiveness to ICI therapy. If pPD-L1$^{adj.}$ is equal or superior to reference value x, e.g. 2.1%, there might be a therapeutic benefit for the patient, if pPD-L1$^{adj.}$ is less than reference value x there might be no benefit.

In Fig. 19 an exemplary calculation for the determination of pPD-L1$^{adj.}$ is depicted. CD62P$^{expr.}$ and PD-L1$^{expr.}$ on the platelet surface are determined. From the CD62P$^{expr.}$ (e.g. 50%) and the PD-L1$^{expr.}$ (e.g. 1.5% = Q3) the correction value pPD-L1$^{corr.}$ is given in the matrix (e.g. 2.53 %). Finally, the correction value is added to the pPD-L1$^{expr.}$ (e.g. pPD-L1$^{expr.}$ + pPD-L1$^{corr.}$ = pPD-L1$^{adj.}$ /1.5% + 2.53 % = 3.03 %). According to step 5 this patient would be assigned into the pPD-L1$^{adj.}$ high group (> 2.1 %) and might thus benefit from ICI.

## 3. Discussion

Human cancers are heterogeneous and biomarkers based on histopathological analyses of single tumor biopsies are often lacking robustness. Histological quantification of intratumoral PD-L1 expression is routinely performed on NSCLC biopsy material as an attempt to predict responses towards immune checkpoint inhibition, however, the correlation between expression levels and the overall response rate (ORR) is limited. In the inventors' present study they show that blood platelets are in frequent contact with lung cancer cells *in vitro* and *in vivo* and take up PD-L1 from the cancer cells in a fibronectin 1, integrin $\alpha5\beta1$ and GPIb$\alpha$ dependent manner. The data provides mechanistic explanation for recent reports describing PD-L1 on platelets from patients suffering from different types of cancers. Interestingly, while it has been a paradigm that platelets degranulate and become inactive after contacting tumor cells, the inventors' data obtained in a live imaging platform suggest that platelets remain active and can re-enter circulation after interaction with tumor cells. Since pPD-L1 was not only detected on the surface of activated platelets but also in resting platelets, it is tempting to speculate on an equilibrium between intracellularly stored pPD-L1 in $\alpha$-granules and cell surface pPD-L1. Indeed, a similar mechanism has been described for the uptake and redistribution of fibrinogen and immunoglobulins.

Importantly, as pPD-L1 was found to inhibit T cell function, it is likely that pPD-L1 plays a distinct role in systemic immunomodulation. Of note, pPD-L1 has recently been described in patients suffering from tumors which were classified as PD-L1 negative in biopsies. The inventors' herein presented data as well as other published studies on tumor heterogeneity suggest that immunohistochemistry-based quantification of protein expression on tissue sections from single biopsies should be interpreted with caution, as protein expression might differ spatially and temporally. Obviously, while the herein presented data suggest a highly efficient uptake of PD-L1 from lung cancer cells into platelets, it does not exclude that some pPD-L1 might be derived from other sources such as endothelial or other non-malignant cell types.

As the total blood volume is circulated up to 1000 times through the body each day, the inventors reasoned that platelets might mirror the collective PD-L1 payload of a tumor and thus might open up venues for novel biomarker strategies. In this regard it is striking that pPD-L1 not only correlated with tumor stage/grade and the occurrence of metastases but was found to be superior in predicting response towards immune checkpoint inhibition when compared to standard histological PD-L1 quantification on tumor biopsies. Since in particular lung cancer represents one of the most frequent and lethal cancers worldwide, further clinical investigation of pPD-L1 as a biomarker in NSCLC does not only hold the promise to unburden the health systems by avoiding costly and unnecessary therapies with ICI but, even more important, will avoid side effects of ICI in patients who would not benefit from this kind of therapy.

Besides the tremendous potential of pPD-L1$^{adj.}$ as a biomarker, the inventors believe that platelet PD-L1 might also represent a potential target for therapeutic intervention. This presumption is supported by the inventors' observation that pPD-L1 in NSCLC patients correlates with the number of T cells in TME and the number of infiltrating T cells. Similar observations in a mouse model support this finding. Along these lines it is tempting to speculate that pPD-L1 might be involved in formation of the premetastatic niche by generating an immunotolerant environment at sites distant from the primary tumor (Fig. 20-21). Inhibition of pPD-L1 could prevent the formation of metastasis and such a concept would warrant the investigation of immune checkpoint blocking antibodies in order to prevent metastasis when tumors with high metastatic risk are treated in a curative intention. Of note, clinical trials investigating the perioperative administration of ICI in NSCLC have reported reduced relapse and metastasis and the herein presented data might offer a mechanistic explanation for the observed results.

Last but not least, as determination of pPD-L1 is highly sensitive and pPD-L1 expression is not found in healthy individuals, pPD-L1 quantification might also be used for early detection of cancer and detection of tumor recurrence.

## Claims

1. A method of predicting a patient's benefit from therapy with an immune checkpoint inhibitor, comprising the steps of:

   1) Providing a platelets-containing sample from the patient;
   2) Determining the expression levels on the platelets' surface of:

      - platelet programmed cell death 1 ligand 1 (pPD-L1) to obtain pPD-L1$^{expr.}$, and
      - a platelet activation marker (A) to obtain A$^{expr.}$;

   3) Determining a correction value pPD-L1$^{corr.}$ as follows:

      - Providing a matrix with $m$ rows and $n$ columns, the elements $a_{ij}$ for the $i$-th row and the $j$-th column, where each row $i$ is assigned to an expression level of A and each column $j$ is assigned to an expression level of pPD-L1,
      - selecting the element $a_{ij}$ from the matrix based on the expression levels determined in step 2 to obtain pPD-L1$^{corr.}$;

   4) Determining an adjusted expression level on the platelet surface of pPD-L1 to obtain pPD-L1$^{adj.}$ as follows:

   $$\text{pPD-L1}^{expr.} + \text{pPD-L1}^{corr.} = \text{pPD-L1}^{adj.};$$

   5) Predicting

      - a therapeutic benefit if pPD-L1$^{adj.} \geq$ reference value x, or
      - no therapeutic benefit if pPD-L1$^{adj.} <$ reference value x.

2. The method of claim 1, wherein A is CD62P and A$^{expr.}$ is CD62P$^{expr.}$.

3. The method of claim 1 or 2, wherein $n$ is 4 resulting in pPD-L1 quartile groups Q1 (very low), Q2 (low), Q3 (high), and Q4 (very high).

4. The method of claim 3, wherein

   - Q1 is assigned to an expression level of pPD-L1 of approx. 0 - 0.3%,
   - Q2 is assigned to an expression level of pPD-L1 of approx. 0.4 - 0.9%,
   - Q3 is assigned to an expression level of pPD-L1 of approx. 1 - 2%,
   - Q4 is assigned to an expression level of pPD-L1 of approx. $\geq$ 2.1%.

5. The method of any of claims 2 to 4, wherein $m$ is 5 resulting in CD62P expression groups E1, E2, E3, E4, and E5.

6. The method of claim 5, wherein

- E1 is assigned to an expression level of CD62P of approx. >80 - 100%,
- E2 is assigned to an expression level of CD62P of approx. >60 - 80%,
- E3 is assigned to an expression level of CD62P of approx. >40 - 60%,
- E4 is assigned to an expression level of CD62P of approx. >20 - 40%,
- E5 is assigned to an expression level of CD62P of approx. 0 - 20%.

7. The method of any of the preceding claims, wherein the elements $a_{ij}$ are as follows:

$a_{11} = 0$; $a_{12} = 0.10$; $a_{13} = 1.10$; $a_{14} = 2.14$;
$a_{21} = 0.01$; $a_{22} = 0.12$; $a_{23} = 1.57$; $a_{24} = 3.50$;
$a_{31} = 0.20$; $a_{32} = 0.81$; $a_{33} = 2.53$; $a_{34} = 4.70$;
$a_{41} = 0.37$; $a_{42} = 1.40$; $a_{43} = 5.43$; $a_{44} = 4.71$;
$a_{51} = 0.60$; $a_{52} = 1.12$; $a_{53} = 2.34$; $a_{54} = 3.14$.

8. The method of any of the preceding claims, wherein x is 2.1%.

9. The method of any of the preceding claims, wherein the expression level of pPD-L1$^{expr.}$ and/or A$^{expr.}$ is determined via flow cytometry, preferably via fluorescence-activated cell sorting (FACS).

10. The method of any of the preceding claims, wherein said platelets-containing sample is a blood sample.

11. The method of any of the preceding claims, wherein said immune checkpoint inhibitor is selected from the group consisting of: pembrolizumab, nivolumab, ipilimumab, tremelimumab, cemiplimab, spartalizumab, atezolizumab, durvalumab, and avelumab.

12. The method of any of the preceding claims, wherein said patient is suffering from non-small cell lung cancer (NSCLC).

13. A method of predicting a cancer patient's probability of survival, comprising the steps of:

1) Providing a platelets-containing sample from the patient;
2) Determining the expression levels on the platelets' surface of:

- platelet programmed cell death 1 ligand 1 (pPD-L1) to obtain pPD-L1$^{expr.}$, and
- a platelet activation marker (A) to obtain A$^{expr.}$;

3) Determining a correction value pPD-L1$^{corr.}$ as follows:

- Providing a matrix with $m$ rows and $n$ columns, the elements $a_{ij}$ for the $i$-th row and the $j$-th column, where each row $i$ is assigned to an expression level of A and each column $j$ is assigned to an expression level of pPD-L1,
- selecting the element $a_{ij}$ from the matrix based on the expression levels determined in step 2 to obtain pPD-L1$^{corr.}$;

4) Determining a matched expression level on the platelet surface of pPD-L1 to obtain pPD-L1$^{adj.}$ as follows:

$$pPD\text{-}L1^{expr.} + pPD\text{-}L1^{corr.} = pPD\text{-}L1^{adj.};$$

5) Predicting

- a low probability of survival if pPD-L1$^{adji}$ $\geq$ reference value x, or
- a high probability of survival if pPD-L1$^{adj.}$ < reference value x.

14. A method for determining in a sample the value of an expression level of a platelet surface protein, said expression level being independent of the activation state of the platelet (p$^{md.}$), said method comprising the following steps:

1. Providing a platelets-containing sample,
2. Determining on said platelets the expression level of:

- a platelet surface protein p to obtain $p^{expr.}$, and
- a platelet activation marker A to obtain $A^{expr.}$;

3. Determining a correction value $p^{corr.}$ as follows:

- Providing a matrix with *m* rows and *n* columns, the the elements $a_{ij}$ for the *i*-th row and the *j*-th column, where each row *i* is assigned to an expression level of said platelet activation marker and each column j is assigned to an expression level of said platelet surface protein,
- selecting the element $a_{ij}$ from the matrix based on $p^{expr.}$ and $A^{expr.}$ determined in step 2 to obtain $p^{corr.}$;

4. Determining $p^{ind.}$ as follows:

$$p^{expr.} + p^{corr.} = p^{ind.}.$$

**Fig. 1**

Fig. 1

Fig. 1

Fig. 1

Fig. 2

**b)** Healthy donors

0.04    0.05    0.05    0.05

PD-L1                                                50 kDa

β-Actin                                              42 kDa

**c)** NSCLC patients

0.31    0.34    0.41    0.21

PD-L1                                                50 kDa

β-Actin                                              42 kDa

UICC staging    II      II      II      II

**d)** Advanced NSCLC patients

0.37    0.47    0.63    0.43

PD-L1                                                50 kDa

β-Actin                                              42 kDa

UICC staging    IV     IV     IV     IV

**e)** 

$P = 0.007$

$P = 0.29$

Rel signal intensity

Healthy donors    NSCLC patients    Advanced NSCLC patients

# Fig. 2

Fig. 3

Fig. 3

Fig. 3

**Fig. 4**

Fig. 4

Fig. 4

**m)**

NCI-H23    HOP-62

**n)**

$P = 0.002$

PLA foci/cell

**o)**

HOP-62

| | | | |
|---|---|---|---|
| Fibronectin 1 | | | 270 kDA |
| α-Tubulin | | | 50 kDA |
| PD-L1-GFP | | | 80 kDA |
| Vinculin | | | 120 kDA |

| | | | |
|---|---|---|---|
| untreated | + | - | - |
| PD-L1-GFP | - | + | + |
| NC siRNA | - | + | - |
| FN1 siRNA | - | - | + |

**p)**

$P = 0.11$
$P < 0.001$
$P < 0.001$

PD-L1 positive PLT (%)

| | | | | |
|---|---|---|---|---|
| untreated | + | - | - | - |
| PD-L1-GFP | - | + | + | + |
| NC siRNA | - | - | + | - |
| FN1 siRNA | - | - | - | + |

**q)**

$P = 0.97$
$P = 0.03$

Ratio (PD-L1-GFP psitive PLT / PD-L1-GFP positive TC)

| | | | | |
|---|---|---|---|---|
| untreated | + | - | - | - |
| PD-L1-GFP | - | + | + | + |
| NC siRNA | - | - | + | - |
| FN1 siRNA | - | - | - | + |

**r)**

control    Vehicle    GPIIbIIIa blocking

Fibronectin 1 coating

IgG    Integrin α5β1 mAb    GPIIbIIIa blocking

Fibronectin 1 coating

**Fig. 4**

Fig. 4

Fig. 5

**Fig. 5**

**i)** Platelets derived from peripheral blood

**i)** Platelets derived from NSCLC patient

# Fig. 5

Fig. 6

Fig. 6

Fig. 6

Fig. 7

Fig. 8

Fig. 8

**Fig. 8**

Fig. 9

Fig. 10

Fig. 10

Fig. 11

Generation of
Platelets

↓

Prior stimualtion

FACS based anaylsis of PD-L1
and CD62P expression

↓

Create quartile
groups

↓

Stimulate
platelets via
TRAP-6

↓

FACS based anaylsis of PD-L1
and CD62P expression

↓

Post stimulation analysis

Calculate Δ pPD-L1
expression change

Developement of
calculation matrix

Fig. 12A

Fig. 12B

Fig. 12

Fig. 12A

Fig. 12A | Fig. 12B

Fig. 12

Fig. 12B

Fig. 13

Fig. 13

Fig. 13

**Fig. 14**

**Fig. 14**

Fig. 15

**e)**

| CT scan (prior treatment) | CT scan I (d 62) | CT scan II (d 114) |
|---|---|---|
| Baseline | PD | PR |

**f)**

Fig. 15

Fig. 16

**Fig. 17**

Fig. 17

**Fig. 18**

**Fig. 19**

Fig. 20

**Fig. 21**

Europäisches
Patentamt

European
Patent Office

Office européen
des brevets

# EUROPEAN SEARCH REPORT

Application Number

EP 21 20 5516

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VERENA ROLFES ET AL: "PD-L1 is expressed on human platelets and is affected by immune checkpoint therapy", ONCOTARGET, vol. 9, no. 44, 1 January 2018 (2018-01-01), pages 27460-27470, XP055703908, DOI: 10.18632/oncotarget.25446 * page 27463, left-hand column, paragraph 1 - page 27465, left-hand column, paragraph 1 * | 1-12,14 | INV. G01N33/574 |
| X | HINTERLEITNER CLEMENS ET AL: "Platelet Programmed Cell Death Ligand 1 (pPDL-1) Is a Prognostic Marker in Advanced Lung Cancer", BLOOD, AMERICAN SOCIETY OF HEMATOLOGY, US, vol. 130, 8 December 2017 (2017-12-08), page 3610, XP086630052, ISSN: 0006-4971, DOI: 10.1182/BLOOD.V130.SUPPL_1.3610.3610 * abstract * | 12 | |
| A | ZHOU YANJUN ET AL: "Platelet-expressed immune checkpoint regulator GITRL in breast cancer", CANCER IMMUNOLOGY IMMUNOTHERAPY, SPRINGER, BERLIN/HEIDELBERG, vol. 70, no. 9, 4 February 2021 (2021-02-04), pages 2483-2496, XP037537481, ISSN: 0340-7004, DOI: 10.1007/S00262-021-02866-Y [retrieved on 2021-02-04] * the whole document * | 1-14 | |

TECHNICAL FIELDS SEARCHED (IPC)

G01N

-/--

The present search report has been drawn up for all claims

| Place of search | Date of completion of the search | Examiner |
|---|---|---|
| The Hague | 7 April 2022 | Wiesner, Martina |

EPO FORM 1503 03.82 (P04C01)

page 1 of 2

## EUROPEAN SEARCH REPORT

**Application Number**

EP 21 20 5516

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAKADA KAZUKI ET AL: "Serum markers associated with treatment response and survival in non-small cell lung cancer patients treated with anti-PD-1 therapy", LUNG CANCER, ELSEVIER, AMSTERDAM, NL, vol. 145, 5 May 2020 (2020-05-05), pages 18-26, XP086156285, ISSN: 0169-5002, DOI: 10.1016/J.LUNGCAN.2020.04.034 [retrieved on 2020-05-05] * the whole document * | 1-14 | |
| A | HERBST ROY S. ET AL: "Atezolizumab for First-Line Treatment of PD-L1-Selected Patients with NSCLC", THE NEW ENGLAND JOURNAL OF MEDICINE, vol. 383, no. 14, 1 October 2020 (2020-10-01), pages 1328-1339, XP055909640, US ISSN: 0028-4793, DOI: 10.1056/NEJMoa1917346 * the whole document * | 1-14 | |

TECHNICAL FIELDS SEARCHED (IPC)

The present search report has been drawn up for all claims

| Place of search | Date of completion of the search | Examiner |
|---|---|---|
| The Hague | 7 April 2022 | Wiesner, Martina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 254170 A

**Non-patent literature cited in the description**

- **TENG F. ; MENG X. ; KONG L. ; YU J.** Progress and challenges of predictive biomarkers of anti PD-1/PD-L1 immunotherapy: A systematic review. *Cancer letters,* 2018, vol. 414, 166-173